# EUROPÄISCHE PATENTSCHRIFT

(11) **EP 1 847 588 B1**
(45) Veröffentlichungstag und Bekanntmachung des Hinweises auf die Patenterteilung: **18.02.2009**
(21) Anmeldenummer: 07105502.4
(22) Anmeldetag: 02.04.2007
(51) Int. Cl.: C11B 9/00, A23L 1/226, A61Q 13/00

(54) **Neue Verwendungen von 4-Methyl-5-hydroxy-hexansäurelacton**
New applications for 4-methyl-5-hydroxy hexane acid lactone
Nouvelles utilisations de 4-méthyl-5-hydroxy-lactone d'acides hexanoïques

(30) Priorität: 19.04.2006 US 792945 P
(43) Veröffentlichungstag der Anmeldung: 24.10.2007
(73) Patentinhaber: Symrise GmbH & Co. KG, 37603 Holzminden (DE)
(72) Erfinder: Kindel, Günter, 37671 Höxter (DE); Wiedwald, Bernd, 37603 Holzminden (DE); Schöning, Axel, 37603 Holzminden (DE); Wöhrle, Ingo, 37603 Holzminden (DE); Loges, Hubert, 37671 Höxter (DE); Lages, Rita, 37619 Bodenwerder (DE)
(74) Vertreter: Eisenführ, Speiser & Partner

(56) Entgegenhaltungen:
- EP-A1- 0 513 627
- JP-A- 60 072 816
- US-A- 4 288 350
- DATABASE FSTA [Online] INTERNATIONAL FOOD INFORMATION SERVICE (IFIS), FRANkFURT-MAIN, DE; XP002448569 Database accession no. 69-2-03-p0272 & HONKANEN E. ET AL: "On the Occurrence of a New Lactone Compound Trans-4-Methyl-4-Hydroxyhexanoic Acid Lactone, In Milk" MILK & DAIRY PRODUCTS, Bd. 22, Nr. 6, 1968, Seiten 2041-2043, ACTA CHEMICA SCANDINAVICA 1968 BIOCHEMICAL RES. INST., HELSINKI, FINLAND

## Beschreibung

Die vorliegende Erfindung betrifft primär die Verwendung von 4-Methyl-delta-hexalacton (4 -Methyl-5-hydroxy-hexansäurelacton oder 5,6-Dimethyltetrahydro-2H-pyran-2-on) zum Vermitteln, Modifizieren und/oder Verstärken bestimmter Geruchs- oder Geschmackseindrücke und daneben bestimmte parfümierte oder aromatisierte Artikel wie Riech- und Aromastoffmischungen (Riech- und Aromastoffkompositionen) umfassend (a) eine (sensorisch wirksame) Menge an 4-Methyl-delta-hexalacton, (b) eine, mehrere oder sämtliche Verbindungen ausgewählt aus der Gruppe bestehend aus (i) Menthol, (ii) (-)-Carvon, (iii) Menthon und (iv) Isomenthon sowie vorzugsweise (c) Nonenolid. Die Erfindung betrifft ferner bestimmte Zubereitungen sowie Verfahren zum Vermitteln, Modifizieren und/oder Verstärken bestimmter Geruchs- oder Geschmackseindrücke sowie zur Herstellung von Riech- oder Aromastoffkompositionen, die 4-Methyl-delta-hexalacton umfassen.

Trotz einer Vielzahl bereits vorhandener Riech- bzw. Aromastoffe besteht in der Parfüm- bzw. Aromenindustrie auch weiterhin ein genereller Bedarf an neuartigen Riech- bzw. Aromastoffen, die über ihre primären, nämlich eigenen geruchlichen bzw. geschmacklichen Eigenschaften hinaus zusätzliche positive Sekundäreigenschaften besitzen, beispielsweise in Mischungen mit anderen Riech- bzw. Aromastoffen.

So besteht ein Bedarf an Riech- bzw. Aromastoffen, die (in Riech- oder Aromastoffkompositionen) interessante Geruchsnoten erzeugen und mit ihren neuartigen bzw. originellen Duft- bzw. Geschmackseigenschaften die Möglichkeiten des Parfümeurs bzw. Flavorists erweitern.

Für die Kreation neuartiger moderner Kompositionen besteht ein ständiger Bedarf an Riech- bzw. Aromastoffen mit besonderen geruchlichen/geschmacklichen Eigenschaften, die geeignet sind, als Bestandteil einer Komposition von neuartigen Parfüms oder Aromen mit komplexem Geruchs- bzw. Geschmackscharakter zu dienen. So werden insbesondere Riech- und Aromastoffe gesucht, die neben einer heuartigen Duft- bzw. Geschmacksnote weitere Noten und Aspekte aufweisen, die ihnen geruchlichen bzw. geschmacklichen Charakter und Komplexität verleihen.

Daher bestand die dieser Erfindung zugrunde liegende Aufgabe im Wesentlichen darin, Riech- bzw. Aromastoffe mit heuartigen (Haupt- oder Neben)Noten zu finden, welche gepaart sind mit weiteren interessanten und originellen geruchlichen bzw. geschmacklichen Eigenschaften, wodurch die gesuchten Riech- bzw. Aromastoffe neuartige und originelle Riech- bzw. Aromastoffkompositionen mit besonderen Noten und Aspekten ermöglichen.

Erfindungsgemäß wird diese Aufgabe primär durch die Verwendung von 4-Methyl-delta-hexalacton als Riech- und/oder Aromastoff zum Vermitteln, Modifizieren und/oder Verstärken einer oder mehrerer Geruchs- oder Geschmackseindrücke aus der Gruppe bestehend aus Heu (nach trockenem Gras und etwas süßlich), Cumarin, Lacton und Mundfülle gelöst. Weitere Aspekte der Erfindung ergeben sich aus den beigefügten Patentansprüchen und der nachfolgenden Beschreibung und betreffen dabei insbesondere parfümierte oder aromatisierte Artikel (insbesondere Riech- oder Aromastoffkompositionen sowie der Ernährung, der Mundpflege oder dem Genuss dienenden Zubereitungen) sowie Verfahren.

Der Erfindung liegt unter anderem die überraschende Erkenntnis zugrunde, dass sich 4-Methyl-delta-hexalacton als Riech- und Aromastoff mit heuartiger Note eignet.

Die Strukturformel von 4-Methyl-5-hydroxy-hexansäurelacton (5,6-Dimethyltetrahydro-2H-pyran-2-on, 4-Methyl-delta-hexalacton) ist nachfolgend durch Formel A wiedergegeben:

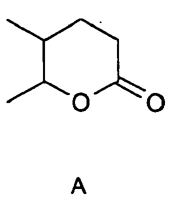

4-Methyl-delta-hexalacton der Formel A kann dabei syn- oder anti-konfiguriert sein, als (R,R)-konfiguriertes Enantiomer, (R,S)-konfiguriertes Enantiomer, (S,R)-konfiguriertes Enantiomer, (S,S)-konfiguriertes Enantiomer oder als beliebiges Gemisch der Enantiomeren, insbesondere als Racemat, oder auch als beliebiges Gemisch der entsprechenden Diastereomeren, vorliegen.

Die Verbindung der Formel A ist bekannt und wurde beispielsweise in Samen des Bockshornklees gefunden (Planta Medica 1985, 533-534) sowie in heissluftgetrockneten Tabakblättern (flue-cured tobacco), siehe beispielsweise Tobacco Science 1976, 20, 125-133 oder auch J. Chromatogr. A 2004, 1040, 1-17. Gemäß Tobacco Science 1976, 20, 125-133 weist die Verbindung der Formel A im Tabakrauch einen süßen, phenolischen und chemischen Geschmack auf. Tabak-haltige Produkte sind keine bevorzugten Produkte im Sinne der vorliegenden Erfindung.

Gemäß J. Agric. Food Chem. 1994, 42, 2344-2348 wurde die Verbindung der Formel A in einigen flüchtigen Fraktionen bestimmter Pilzkulturen (Basidiomyceten) gefunden. Der durch GC/Olfaktometrie ermittelte Geruch des 4-Methyl-delta-hexalactons wurde als fruchtig beschrieben.

In dem jüngst erschienenen Artikel Flavour & Fragrance Journal 2006, 21, 193-197 werden folgende Geruchsbeschreibungen von 5-Methyl-delta-lactonen angegeben, welche in der Lactonfraktion eines kommerziell erhältlichen Süßholzextraktes (Lakritz, *Glycyrrhia glabra* L.) gefunden wurden:
5-Methyl-5-heptanolid: Heu, Cumarin, Pferdestall, animalisch und krautig;
5-Methyl-5-octanolid: fauliges Wasser, phenolisch, holzig, fruchtig und pilzig;
5-Methyl-5-nonanolid: lactonig, getrocknete Kräuter, Heu, fruchtig, süß und Kokosnuss;
5-Methyl-5-decanolid: fauliges Wasser, leicht lactonig, krautig, holzig, und milchig.

EP 0 513 627 beschreibt die delta-Lactone 4-Methyl-5-octanolid bis 4-Methyl-5-tridecanolid allgemein als Riech- und Geschmackstoffe. So wird 5-Methyl-6-pentyl-tetrahydro-alpha-pyron (4-Methyl-5-decanolid) als Riechstoff mit einem Geruch beschrieben, der an bestimmte Blütendüfte und gleichzeitig an Caramel, Kondensmilch und Kokos, insbesondere Kokosmilch, erinnert. 4-Methyl-5-nonanolid weist laut EP 0 513 627 einen Geruch von Kokos und Sellerie auf.

Gemäß JP 60-72816 A kann die Verbindung der Formel A als Arzneimittel zur Behandlung von Schlafstörungen oder von psychoneurotischen Krankheiten eingesetzt werden. In diesem Dokument ist auch die Herstellung der Verbindung A beschrieben.

In Anlehnung an die EP 0 513 627 kann die Verbindung der Formel A auch durch Bayer-Villiger-Oxidation mit Persäuren, wie beispielsweise Peressigsäure, aus 2,3-Dimethylcyclopentanon hergestellt werden, welches wiederum durch Hydrierung aus 2,3-Dimethylcyclopentenon erhältlich ist.

Cumarin (2H-1-Benzopyran-2-on) ist der wesentliche Bestandteil des Geruchs und Geschmacks des Waldmeisters (*Asperula odorata, Galium odoratum*). Cumarin weist unter anderem eine heuartige Note auf und war insbesondere in früheren Zeiten ein häufig eingesetzter Riech- und Aromastoff. Cumarin führt jedoch insbesondere in höheren Dosen zu gesundheitlichen Beeinträchtigungen; aus toxikologischen Gründen wäre es daher wünschenswert, eine alternative Verbindung für Cumarin zu finden.

Die Tatsache, dass das erfindungsgemäß einzusetzende 4-Methyl-delta-hexalacton einen heuartigen, cumarinigen Geruch- bzw. Geschmack hervorzurufen vermag ist überraschend. Dabei ist zu erwähnen, dass die heuartige Note des 4-Methyl-delta-hexalactons merklich stärker ausgeprägt ist als der cumarinige Aspekt.

4-Methyl-delta-hexalacton vermag, insbesondere in Kombination mit einer (d.h. einer einzelnen der nachfolgend aufgeführten Verbindungen (i), (ii), (iii) oder (iv)), mehreren oder sämtlichen Verbindungen ausgewählt aus der Gruppe bestehend aus (i) Menthol, (ii) (-)-Carvon, (iii) Menthon und (iv) Isomenthon, je nach Einsatzkonzentration, einen vielfältigen Geruchs- und Geschmackseindruck der Noten Heu, Cumarin, Lacton und/oder Mundfülle zu bewirken.

4-Methyl-delta-hexalacton der Formel A kann auf Grund der von ihm vermittelten Geruchs- und Geschmackseindrücke insbesondere eingesetzt werden bei der Herstellung von Aromen und Ölen, die in ihren sensorischen (geruchlichen und/oder geschmacklichen) Eigenschaften sowie vorzugsweise in ihrer Konsistenz natürlichen Pfefferminz- und/oder Spearmintaromen bzw. Pfefferminz- und/oder Spearmintölen äußerst ähnlich sind. Man spricht in diesem Zusammenhang von sensorischer Rekonstitution, Rekomposition oder Nachstellung der natürlichen Aromen bzw. Öle.

Die (sensorische) Rekonstitution (Rekomposition, Nachstellung) eines natürlichen Pfefferminz- und/oder Spearmintöls ist beispielsweise von Interesse, weil solche (sensorisch) rekonstituierten, rekomponierten bzw. nachgestellten Öle im Gegensatz zu ihren natürlichen Vorbildern, bei denen es sich um mittels Wasserdampfdestillation gewonnene etherische Öle handelt, keinen (sensorischen) Qualitätsschwankungen unterliegen und in ihren (sensorischen) Eigenschaften nicht von der Ernte, dem Anbaugebiet und/oder dem Gewinnungsprozess abhängen.

Bei der Rekonstitution, Rekomposition bzw. Nachstellung natürlicher Pfefferminz- und/oder Spearmintaromen bzw. natürlicher Pfefferminz- und/oder Spearmintöle wird neben dem 4-Methyl-delta-hexalacton der Formel A vorzugsweise auch das gamma-Lacton Nonenolid (2-Nonen-4-olid; 4-Hydroxy-2-nonensäurelacton; 5-Pentyl-5H-furan-2-on) der Formel C eingesetzt, siehe dazu weiter unten im Detail.

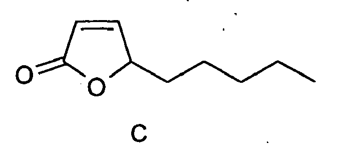

Gemäß einer bevorzugten Ausgestaltung der erfindungsgemäßen Verwendung wird 4-Methyl-delta-hexalacton (vorzugsweise gemeinsam mit Nonenolid der Formel C) zum Vermitteln, Modifizieren und/oder Verstärken einer oder mehrerer Geruchs- oder Geschmackseindrücke aus der Gruppe Heu und Cumarin eingesetzt, wobei in dem resultierenden 4-Methyl-delta-hexalacton-haltigen Produkt (Artikel) die Verbindung Cumarin selbst nicht enthalten ist, und vorzugsweise auch keine Fraktion eines natürlichen Öls oder Aromas enthalten ist, die selbst eine heuartige, cumarinige Note besitzt.

Unter natürlichen Pfefferminzölen, welche unter Verwendung von 4-Methyl-delta-hexalacton (sensorisch) rekonstituiert werden können, werden im Speziellen die etherischen (d.h. mittels Wasserdampfdestillation gewonnenen) Öle bestimmter Mentha-Spezies verstanden, insbesondere aus *Mentha arvensis* (Ackerminze, US-sprachlich auch cornmint genannt) und aus *Mentha piperita* (US-sprachlich peppermint genannt), diese schließen *Mentha piperita* Öle mit regionalen Herkunftsbezeichnungen spezieller Anbaugebiete wie Willamette, Yakima und Madras ein.

Die besagten natürlichen Pfefferminzöle weisen eine heuartige, cumarinige Note auf, welche den bislang bekannten (sensorisch) rekonstituierten, rekomponierten oder nachgestellten Pfefferminzölen fehlt. Die heuartige, cumarinige Note natürlicher Pfefferminzöle rührt allerdings nicht vom Cumarin, da diese kein Cumarin enthalten, sondern wohl von anderen, teilweise nicht identifizierten und in Pfefferminzölen nur in Spuren vorhandenen, Verbindungen.

Unter natürlichen Spearmintölen (Krauseminzölen), welche unter Verwendung von 4-Methyl-delta-hexalacton (sensorisch) rekonstituiert werden können, werden im Speziellen die etherischen Öle aus *Mentha cardiaca* oder *Mentha spicata* verstanden.

Die Erfindung betrifft ferner parfümierte oder aromatisierte Artikel umfassend oder bestehend aus (a) 4-Methyl-delta-hexalacton, (b) einer, mehreren oder sämtlichen Verbindungen ausgewählt aus der Gruppe bestehend aus (i) Menthol, (ii) (-)-Carvon, (iii) Menthon, (iv) Isomenthon sowie vorzugsweise (c) Nonenolid und/oder (d) Menthylacetat.

4-Methyl-delta-hexalacton verleiht einem entsprechend parfümierten oder aromatisierten Artikel eine besondere Natürlichkeit und/oder Authentizität.

Vorzugsweise ist ein parfümierter oder aromatisierter erfindungsgemäßer Artikel dabei ausgewählt aus der Gruppe bestehend aus:
- Riech- oder Aromastoffkomposition, vorzugsweise Pfefferminz- oder Krauseminzaromastoffkomposition,
- der Ernährung dienende Zubereitung, vorzugsweise verzehrfertiges oder nicht verzehrfertiges Lebensmittel,
- der Mundpflege dienende Zubereitung und
- dem Genuss dienende Zubereitung.

In den erfindungsgemäßen parfümierten oder aromatisierten Artikeln, insbesondere den bevorzugten erfindungsgemäßen Artikeln aus der vorstehend genannten Gruppe, ist vorzugsweise eine Menge an 4-Methyl-delta-hexalacton enthalten, die ausreicht, um einen oder mehrere Geruchs- oder Geschmackseindrücke aus der Gruppe Heu, Cumarin, Lacton und Mundfülle zu vermitteln, zu modifizieren und/oder zu verstärken. Vorzugsweise umfassen die erfindungsgemäßen Artikel kein Cumarin und keine Fraktion eines natürlichen Öls oder Aromas, die selbst eine heuartige, cumarinige Note besitzt.

L-Menthol hat einen einzigartigen erfrischenden Geschmack, einen minzigen Geruch und eine stark wirkende Kühlung auf Haut und Schleimhaut. Es wird beispielsweise bei der Mundpflege, in kosmetischen und pharmazeutischen Präparaten, im Tabak und in Süßwaren verwendet, wie z.B. in Perfumer&Flavorist, Vol. 13, October-November 1988, S. 37 beschrieben. L-Menthol ist der Hauptbestandteil der Pfefferminzöle aus *Mentha arvensis* (Gehalt: etwa 70 - 80 Gew.-%) und *Mentha piperita* (Gehalt: etwa 50 - 60 Gew.-%).

Im Sinne der Erfindung kann das vorzugsweise in erfindungsgemäßen Artikeln eingesetzte Menthol d-Menthol, I-Menthol oder eine beliebige Mischung hiervon sein, bevorzugt sind I-Menthol, d-Menthol und racemisches Menthol, insbesondere bevorzugt ist I-Menthol, da I-Menthol die oben genannten sensorischen Eigenschaften aufweist welche für die (sensorische) Rekonstitution von Pfefferminzölen sehr vorteilhaft sind.

Es können auch beliebige Mischungen von synthetischem und natürlichem Menthol sowie von racemischem und enantiomerenreinem Menthol eingesetzt werden.

Das erfindungsgemäß vorzugsweise eingesetzte Menthol kann synthetischen oder natürlichen Ursprungs sein, bevorzugt ist jedoch der Einsatz synthetischen Menthols.

(-)-Carvon ist der Hauptbestandteil des Krauseminzöls (Spearmintöl, Gehalt: etwa 70 - 80 Gew.-%) und hat einen krautigen, krauseminzigen Geruch und Geschmack. Das erfindungsgemäß vorzugsweise eingesetzte (-)-Carvon kann dabei synthetischen oder natürlichen Ursprungs sein.

Menthon und Isomenthon (beide können unabhängig voneinander als (+) und/oder (-) Enantiomer vorliegen) kommen in vielen etherischen Ölen vor, in Pfefferminzölen von *Mentha*-Spezies können sie sogar mit mehr als 50 Gew.-% enthalten sein. Menthon weist einen typischen kräftigen minzigen Geruch und Geschmack auf und wirkt kühlend, Isomenthon ist minzig, camphrig und kühlend, dabei etwas dumpfer als Menthon.

Im Sinne der Erfindung kann das vorzugsweise eingesetzte Menthon (+)-Menthon, (-)-Menthon sowie eine beliebige Mischung hiervon sein, bevorzugt sind (+)-Menthon, (-)-Menthon und racemisches Menthon, insbesondere bevorzugt ist (-)-Menthon, da dieses für die Herstellung nicht-natürlicher oder synthetischer Pfefferminzöle bzw. -aromen und die (sensorische) Rekonstitution natürlicher Pfefferminzöle sehr vorteilhaft ist.

Im Sinne der Erfindung kann das eingesetzte Isomenthon (+)-Isomenthon, (-)-Isomenthon sowie eine beliebige Mischung hiervon sein, bevorzugt sind (+)-Isomenthon, (-)-Isomenthon und racemisches Isomenthon, insbesondere bevorzugt ist (+)-Isomenthon, da dieses für die Herstellung nicht- natürlicher oder synthetischer Pfefferminzöle bzw. -aromen und die (sensorische) Rekonstitution natürlicher Pfefferminzöle sehr vorteilhaft ist.

Aus dem Vorgesagten ergibt sich, dass das erfindungsgemäß einzusetzende 4-Methyl-delta-hexalacton insbesondere dann zum Vermitteln, Modifizieren und/oder Verstärken der genannten Geruchs- oder Geschmackseindrücke befähigt ist, wenn es in Mischungen mit einer, mehreren oder sämtlichen Verbindungen ausgewählt aus der Gruppe bestehend aus (i) Menthol, (ii) (-)-Carvon, (iii) Menthon und (iv) Isomenthon vorliegt. 4-Methyl-delta-hexalacton ist deshalb besonders geeignet zur Einarbeitung in Riech- oder Aromastoffkompositionen, vorzugsweise Pefferminz- und/oder Krauseminzaromakompositionen, sowie in der Ernährung, der Mundpflege oder dem Genuss dienende Zubereitungen mit Pfefferminz- oder Krauseminz-Geruchs- oder -Geschmacksnoten.

Diese Verwendbarkeit des 4-Methyl-delta-hexalacton ist insbesondere deshalb bemerkenswert, weil dessen weiter oben angegebenen bekannten Geruchs- und Geschmackseindrücke (süß, phenolisch, chemisch; fruchtig; siehe insoweit die obigen Dokumente) in Kombination mit einer, mehreren oder sämtlichen Verbindungen der Gruppe bestehend aus (i) Menthol, (ii) (-)-Carvon, (iii) Menthon und (iv) Isomenthon nicht nur weitgehend oder vollständig unterdrückt werden, sondern statt dessen die gesuchte und erwünschte heuartige bzw. cumarinige Note auftritt. Darüberhinaus weist 4-Methyl-delta-hexalacton einen niedrigen sensorischen Schwellenwert auf, so dass es bereits in sehr geringer Dosierung wahrnehmbar ist und, eingearbeitet in eine Riech- oder Aromastoffkomposition, diese signifikant beeinflussen kann (siehe hierzu auch die Ausführungen weiter unten).

Die nachfolgende Tabelle 1 verdeutlicht die beobachteten sensorischen Effekte hinsichtlich einer heuartigen bzw. cumarinigen Note in Mischungen von einer oder mehreren Substanzen der Gruppe bestehend aus (i) Menthol, (ii) (-)-Carvon, (iii) Menthon und (iv) Isomenthon und 4-Methyl-delta-hexalacton der Formel A. Die Verkostung der erfindungsgemäßen Aromen T1 bis T6 gemäß Tabelle 1 erfolgte bei einer jeweiligen Dosierung von 0,2 Gew.-% in einer 5 Gew.-%igen wässrigen Zuckerlösung (Saccharoselösung).

**Tabelle 1 (alle Anteile sind in Gew.-% angegeben)**

| | **T1** | **T2** | **T3** | **T4** | **T5** | **T6** |
|---|---|---|---|---|---|---|
| 4-Methyl-delta-hexalacton der Formel A | 0,10 | 0,20 | 0,15 | 0,1 | 0,15 | 0,1 |
| (i) Menthol | 99,9 | | | 50,0 | 50,0 | 65,0 |
| (ii) (-)-Carvon | | 99,8 | | 49,9 | | |
| (iii) Menthon | | | 99,85 | | 25,0 | 23,0 |
| (iv) Isomenthon | | | | | 24,85 | 11,8 |
| (c) Nonenolid | | | | | | 0,1 |
| Bewertung der Noten Heu und Cumarin | + | + | + | ++ | +++ | ++++ |

| | | | | | | |
|---|---|---|---|---|---|---|
| +: merkliche heuartige bzw. cumarinige Note; ++: mittlere heuartige bzw. cumarinige Note; +++: ausgeprägte heuartige bzw. cumarinige Note; ++++: kräftige heuartige bzw. cumarinige Note. | | | | | | |

Die in Tabelle 1 gezeigten Ergebnisse belegen, dass in Mischungen von 4-Methyl-delta-hexalacton der Formel A und einer einzelnen Substanz der Gruppe bestehend aus (i) Menthol, (ii) (-)-Carvon, (iii) Menthon und (iv) Isomenthon bereits eine heuartige bzw. cumarinige Note auftritt. Deutlicher tritt eine heuartige bzw. cumarinige Note auf, wenn zwei oder mehr Substanzen der Gruppe bestehend aus (i) Menthol, (ii) (-)-Carvon, (iii) Menthon und (iv) Isomenthon mit 4-Methyl-delta-hexalacton der Formel A kombiniert werden, wobei ferner auch eine Abrundung des Geruchs- bzw. Geschmacksprofils beobachtet werden kann.

Für das erfindungsgemäß einzusetzende 4-Methyl-delta-hexalacton wurden bei der Verkostung einer Mischung bestehend aus 5 Gew.-%iger wässriger Zuckerlösung und Pfefferminzaroma (Dosierung: 5000 ppm = 0,5 Gew.-%, das Pfefferminzaroma enthielt (i) I-Menthol zu 62 Gew.-%, 5% Menthylacetat (Bestandteil d)) und in Summe (iii) (-)-Menthon und (iv) (+)-Isomenthon zu 23 Gew.-% sowie weitere Bestandteile, die zum Geschmackseindruck keinen wesentlichen Beitrag leisten) die folgenden geschmacklichen Eigenschaften gefunden:
bei einer Konzentration an 4-Methyl-delta-hexalacton von 15 ppm (bezogen auf die Gesamtmenge der verkosteten Mischung):
   die Noten Heu und Cumarin werden beim Einsatz nicht natürlichen Pfefferminzöls vermittelt (analog wurden bei Verwendung natürlichen Pfefferminzöls, z.B. mit einem Anteil an I-Menthol von 70 Gew.-%, die Noten Heu und Cumarin verstärkt). Dieser Effekt dominiert im Bereich von etwa 5 - 20 ppm.

Bei einer Konzentration an 4-Methyl-delta-hexalacton von 25 ppm: Lacton-Note Dieser Effekt dominiert im Bereich von etwa 20 - 30 ppm.

Bei einer Konzentration an 4-Methyl-delta-hexalacton von 40 ppm: Mundfülle Dieser Effekt dominiert oberhalb von 30 ppm, d.h. im Bereich von etwa 30 - 100 ppm.

Bei noch höherer Dosierung, etwa im Bereich von 1 Gew.-% und höher, bezogen auf die Aromakomposition, treten verstärkt fruchtige Noten auf.

Die vorstehend genannten geruchlichen bzw. geschmacklichen Effekte gehen in den Grenzbereichen der angegebenen Konzentrationen jeweils fließend ineinander über. Vorstehend wurden Konzentrationsbereiche angegeben, in denen der jeweils angegebene geruchliche bzw. geschmackliche Effekt eindeutig dominiert.

Unter dem Geschmackseindruck "Mundfülle" wird vorliegend der mundfüllende Geschmackseindruck von Cremigkeit, Sahnigkeit und Milchfettigkeit verstanden.

Das erfindungsgemäß einzusetzende 4-Methyl-delta-hexalacton der Formel A kann auch zur Reduzierung der Bitterkeit bitter schmeckender Substanzen eingesetzt werden. Bei Einsatz von 4-Methyl-delta-hexalacton wurde in Artikeln, die eine bittere Geschmacksnote aufweisen, in manchen Fällen eine Reduzierung der Bitterkeit bereits bei Zugabe geringer Mengen von 4-Methyl-delta-hexalacton der Formel A festgestellt, beispielsweise bei Kaugummis, deren Kaugummibase als bitter schmeckend empfunden wurde, oder bestimmten Süßstoff-haltigen Artikeln, die auf Grund der Anwesenheit bestimmter Süßstoffe mit bitterem Nachgeschmack insgesamt als bitterschmeckend beurteilt werden. Ebenfalls konnte eine Reduzierung der Bitterkeit bei Herstellung bestimmter erfindungsgemäßer parfümierter oder aromatisierter Artikel festgestellt werden, die das in höherer Konzentration bitter schmeckende Menthol umfassten. Die Anwesenheit von 4-Methyl-delta-hexalacton neben Menthol unterdrückte dessen bitterschmeckende Wirkung.

In erfindungsgemäßen Riech- oder Aromastoffkompositionen ist vorzugsweise eine Menge an 4-Methyl-delta-hexalacton der Formel A im Bereich von 50 bis 8000 ppm, bevorzugt 100 bis 5000 ppm und besonders bevorzugt 250 bis 2000 ppm enthalten, bezogen auf die Gesamtmenge der Riech- oder Aromastoffkomposition.

Vorzugsweise ist das Gewichtsverhältnis von 4-Methyl-delta-hexalacton der Formel A zur Gesamtmasse der Verbindungen der Gruppe bestehend aus (i) Menthol, (ii) (-)-Carvon, (iii) Menthon und (iv) Isomenthon kleiner als 1 : 1, bevorzugt liegt das Gewichtsverhältnis im Bereich von 1 : 50 bis 1 : 2500, besonders bevorzugt im Bereich von 1 : 100 bis 1 : 1200.

Bevorzugt sind erfindungsgemäße Riech- oder Aromastoffkompositionen oder der Ernährung, der Mundpflege oder dem Genuss dienende Zubereitungen (insbesondere gemäß einer der vorstehend als bevorzugt bezeichneten Ausgestaltungen), die eine Menge an einer oder mehren Verbindungen ausgewählt aus der Gruppe bestehend aus (i) Menthol, (ii) (-)-Carvon, (iii) Menthon und (iv) Isomenthon enthalten, die ausreicht, um in der Riech- oder Aromastoffkomposition oder der Zubereitung eine dominierende Pefferminz- und/oder Krauseminz-Geruchs- oder Geschmacksnote zu erzeugen. In solchen Riech- oder Aromastoffkompositionen oder Zubereitungen ist - wie ausgeführt - eine Menge an 4-Methyl-delta-hexalacton enthalten, die ausreicht, um einen oder mehrere Geruchs- oder Geschmackseindrücke aus der Gruppe Heu, Cumarin, Lacton und Mundfülle zu vermitteln, zu modifizieren und/oder zu verstärken. Die Substanz Cumarin ist vorzugsweise in einem solchen erfindungsgemäßen Artikel nicht vorhanden, und vorzugsweise umfasst ein solcher Artikel auch kein natürliches Pfefferminz- oder Krauseminzöl und kein natürliches Pfefferminz- oder Krauseminzaroma. Insbesondere ist aber vorzugsweise zumindest keine Fraktion eines natürlichen Öls oder Aromas vorhanden, wie sie in den besagten natürlichen Ölen oder Aromen für die heuartige, cumarinige Note verantwortlich ist.

In bevorzugten erfindungsgemäßen Riech- oder Aromastoffkompositionen liegen die Konzentrationen der relevanten oben genannten Bestandteile vorzugsweise in bestimmten Bereichen. Insbesondere bevorzugt sind erfindungsgemäße Riech- oder Aromakompositionen (vorzugsweise gemäß einer der vorstehend angegebenen bevorzugten Ausgestaltungen), umfassend:
(a) 4-Methyl-delta-hexalacton in einer Menge von vorzugsweise 50 bis 8000 ppm, bevorzugt 100 bis 5000 ppm und besonders bevorzugt 250 bis 2000 ppm, sowie
(b)
   (i) Menthol, vorzugsweise I-Menthol, in einer Menge von 1 bis 80 Gew.-%, bevorzugt 5 bis 70 Gew.-%,
      und/oder
   (ii) (-)-Carvon in einer Menge von 1 bis 80 Gew.-%, bevorzugt 5 bis 80 Gew.-%, und/oder
      (iii) Menthon, vorzugsweise (-)-Menthon,
         und/oder
      (iv) Isomenthon, vorzugsweise (+)-Isomenthon, in einer Gesamtmenge von 0,5 bis 60 Gew.-%, bevorzugt 1 bis 50 Gew.-%,
      sowie vorzugsweise
(c) Nonenolid in einer Menge von 50 bis 8000 ppm, bevorzugt 100 bis 5000 ppm und besonders bevorzugt 250 bis 2000 ppm, und/oder
(d) Menthylacetat, vorzugsweise (-)-Menthylacetat in einer Menge von 1 bis 20 Gew.-%, bevorzugt 2 bis 12 Gew.-%, besonders bevorzugt 3 bis 10 Gew.-%,
wobei die Mengenangaben jeweils bezogen sind auf die Gesamtmenge der Riech- oder Aromastoffkomposition.

Der Anteil an (i) Menthol liegt hierbei in rekonstituierten (rekombinierten, nachgestellten) Pfefferminzaromen und Pfefferminzölen vorzugsweise im Bereich von 30 bis 60 Gew.-%.

Der Gehalt an (-)-Carvon liegt hierbei in rekonstituierten (rekombinierten, nachgestellten) Spearmintaromen und Spearmintölen vorzugsweise im Bereich von 30 bis 70 Gew.-%.

Der Gesamtanteil an Menthon und Isomenthon liegt hierbei in rekonstituierten (rekombinierten, nachgestellten) Pfefferminzaromen und Pfefferminzölen vorzugsweise im Bereich von 5 bis 40 Gew.-%.

Die Strukturformeln der besonders bevorzugten Verbindungen der Gruppe (b) sind im Folgenden dargestellt:

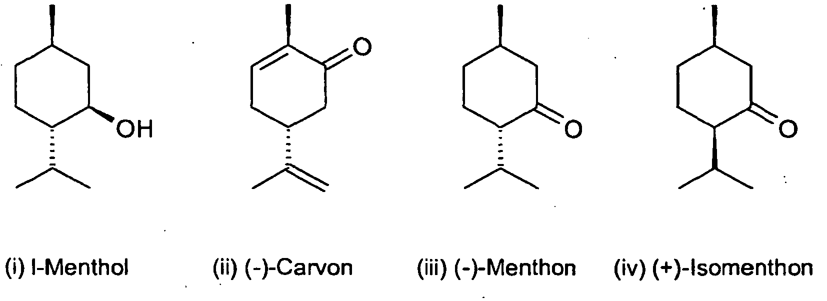

Die Strukturformel des erfindungsgemäß besonders bevorzugt als Komponente (d) einzusetzenden (-)-Menthylacetat (I-Menthylacetat) ist in der nachfolgenden Formel D wiedergegeben.

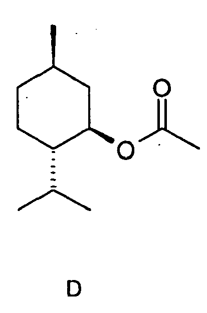

Die bevorzugte Komponente (d) (-)-Menthylacetat (der Formel D) weist einen frisch-fruchtigen, pfefferminzigen Geruch auf und komplettiert insbesondere bei rekonstituierten (rekombinierten, nachgestellten) Pfefferminzaromen und Pfefferminzölen das Geruchs- und Aromaprofil.

Auch in den erfindungsgemäßen der Ernährung, der Mundpflege oder dem Genuss dienenden Zubereitungen (wie oben ausgeführt) sind bestimmte Konzentrationen von 4-Methyl-delta-hexalacton bzw. den erfindungsgemäßen Riech- oder Aromastoffkompositionen bevorzugt. Besonders bevorzugt sind erfindungsgemäße Zubereitungen umfassend
- 4-Methyl-delta-hexalacton in einer Menge im Bereich von 0,05 bis 80 ppm, bevorzugt im Bereich von 0,1 bis 50 ppm und besonders bevorzugt im Bereich von 0,25 bis 20 ppm
   oder
- eine erfindungsgemäße Riech- oder Aromastoffkomposition (vorzugsweise in einer bevorzugten Ausgestaltung) in einer Menge von 0,05 bis (in bestimmten Fällen) 50 Gew.-%, bevorzugt im Bereich von 0,05 bis 6 Gew.-%, vorzugsweise im Bereich von 0,1 bis 3 Gew.-%,
bezogen auf das Gesamtgewicht der Zubereitung.

Die vorstehend genannten Konzentrationsangaben für erfindungsgemäße Zubereitungen betreffen insbesondere gebrauchsfertige Zubereitungen, d.h. direkt vom Endverbraucher einsetzbare Zubereitungen. Gebrauchsfertig sind insbesondere verzehrfertige Lebensmittel wie z. B. verzehrfertige Süßwaren oder verzehrfertige Getränke. Gebrauchsfertig sind aber auch Mundpflegeprodukte (Mundhygieneprodukte), welche direkt vom Konsumenten eingesetzt werden können

In gebrauchsfertigen Zubereitungen, insbesondere bei Süßwaren (wie Kaugummis und Karamellen) oder bei Mundhygieneprodukten (wie Zahnpasten, - cremes und -gelen), liegt der Anteil einer erfindungsgemäßen 4-Methyl-delta-hexalacton der Formel A enthaltenden Riech- oder Aromastoffkomposition vorzugsweise im Bereich von 0,05 bis 6 Gew.-%, bevorzugt im Bereich von 0,1 bis 3 Gew.-%, bezogen auf das Gesamtgewicht der gebrauchsfertigen Zubereitung.

In verzehrfertigen alkoholischen oder nicht-alkoholischen Getränken im Sinne der Erfindung liegt der Anteil einer erfindungsgemäßen 4-Methyl-delta-hexalacton der Formel A enthaltenden Riech- oder Aromastoffkomposition vorzugsweise im Bereich von 3 bis 100 ppm, bevorzugt im Bereich von 5 bis 50 ppm, bezogen auf das Gesamtgewicht der verzehrfertigen Zubereitung.

Sofern es sich bei der gebrauchsfertigen Zubereitung allerdings um eine zum Direktverzehr geeignete Kapsel handelt (siehe Beispiel F10 unten) kann der Anteil der einzusetzenden erfindungsgemäßen 4-Methyl-delta-hexalacton enthaltenden Riech- oder Aromastoffkomposition deutlich höher liegen und bis zu 50 Gew.-% betragen.

Die erfindungsgemäßen der Ernährung, der Mundpflege oder dem Genuss dienenden Zubereitungen sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel) oder wieder aus der Mundhöhle entfernt zu werden (z.B. Kaugummis oder Zahnpasta). Es versteht sich, dass der Einsatz von 4-Methyl-delta-hexalacton bzw. den erfindungsgemäßen Riech- oder Aromastoffkompositionen für jede Art solcher Produkte vorgesehen ist. Zu diesen Produkten gehören dabei sämtliche Stoffe oder Erzeugnisse, die dazu bestimmt sind, in verarbeitetem, teilweise verarbeitetem oder unverarbeitetem Zustand vom Menschen aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Es versteht sich, dass 4-Methyl-delta-hexalacton sowie die erfindungsgemäßen Riech- oder Aromastoffkompositionen insbesondere in Lebensmitteln eingesetzt werden können. Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Insbesondere wird 4-Methyl-delta-hexalacton bzw. eine erfindungsgemäße Riech- oder Aromastoffkomposition in verzehrfertigen Lebensmitteln eingesetzt. Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme, aber auch Kaugummis oder Hartkaramellen, zählen zu den verzehrfertigen Lebensmitteln.

4-Methyl-delta-hexalacton sowie erfindungsgemäße Riech- oder Aromastoffkompositionen können auch in Lebensmittel-Halbfertigwaren eingesetzt werden. Der Begriff Lebensmittel-Halbfertigware bezieht sich hierbei auf Lebensmittel, die dazu bestimmt sind, erst im weiter verarbeiteteten Zustand, nach Hinzufügen von für den sensorischen Eindruck (mit)entscheidenden Aroma- oder Geschmacksstoffen verzehrt zu werden.

Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind Cremes, Gele, Pasten, Schäume, Emulsionen, Suspensionen, Areosole, Sprays als auch Kapseln, Granulate, Pastillen, Tabletten, Bonbons oder Kaugummis, ohne dass diese Aufzählung für die Zwecke dieser Erfindung limitierend verstanden werden soll.

Bevorzugte verzehrfertige alkoholische oder nicht-alkoholische Getränke im Sinne der Erfindung sind (vorzugsweise kohlensäurefreie) nicht-alkoholische Erfrischungsgetränke oder alkoholische Liköre.

In einem verzehrfertigen nicht-alkoholischen Getränk im Sinne der Erfindung liegt der Anteil einer erfindungsgemäßen 4-Methyl-delta-hexalacton der Formel A enthaltenden Riech- oder Aromastoffkomposition vorzugsweise im Bereich von 3 bis 30 ppm (entsprechend etwa 0,3 bis 3 g pro 100 Litern Getränk), bevorzugt im Bereich von 5 bis 15 ppm (entsprechend etwa 0,5 bis 1,5 g pro 100 Litern Getränk), bezogen auf das Gesamtgewicht der verzehrfertigen Zubereitung.

Verzehrfertige nicht-alkoholische Getränke im Sinne der Erfindung weisen vorzugsweise einen Zuckergehalt (Saccharosegehalt) bzw. einen Gehalt an Glucosesirup im Bereich von 8 bis 15 Gew.-%, bevorzugt im Bereich von 9 bis 13 Gew.-%, auf, bezogen auf das verzehrfertige nicht-alkoholische Getränk.

In einem verzehrfertigen alkoholischen Getränk im Sinne der Erfindung liegt der Anteil einer erfindungsgemäßen 4-Methyl-delta-hexalacton der Formel A enthaltenden Riech- oder Aromastoffkomposition vorzugsweise im Bereich von 10 bis 100 ppm (entsprechend etwa 1 bis 10 g pro 100 Litern Getränk), bevorzugt im Bereich von 25 bis 80 ppm (entsprechend etwa 2,5 bis 8 g pro 100 Litern Getränk), bezogen auf das Gesamtgewicht der verzehrfertigen Zubereitung. Verzehrfertige Getränke im Sinne der Erfindung sind vorzugsweise Liköre und weisen vorzugsweise einen Zuckergehalt (Saccharosegehalt) bzw. einen Gehalt an Glucosesirup im Bereich von 10 bis 25 Gew.-%, bevorzugt im Bereich von 15 bis 20 Gew.-%, auf, bezogen auf das verzehrfertige alkoholische Getränk. Der Alkoholgehalt liegt dabei vorzugsweise im Bereich von 12 bis 25 vol.-%, vorzugsweise im Bereich von 15 bis 20 vol.-%, bezogen auf das verzehrfertige alkoholische Getränk.

Eine bevorzugte erfindungsgemäße gebrauchsfertige Zubereitung ist (A) eine verzehrfertige und dabei zuckerfreie, zuckerreduzierte oder zuckerhaltige Süßware, insbesondere in Form von Schokolade, gefüllten Schokolade (beispielsweise mit aromatisierter Fondant-Masse, z.B. des Typs After-Eight), Schokoladenriegelprodukt, Fruchtgummi, Hart- oder Weichkaramelle, Kaubonbon, Zuckerperle, Lutscher, Kapsel (vorzugsweise nahtlose Kapsel, bevorzugt zum Direktverzehr, vorzugsweise mit einer Umhüllung basierend auf Gelatine und/oder Alginat), Kaugummi (z.B. in Form von Streifen, Komprimaten, Pellets, Kissen, Kugeln, Hohlkugeln), (B) ein Mundpflegeprodukt (Mundhygieneprodukt), insbesondere in Form von Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide, oder Zahnpflegekaugummi oder (C) ein alkoholisches oder alkoholfreies Getränk.

Unter die Begriffe "der Ernährung dienende Zubereitung" bzw. "dem Genuss dienende Zubereitung" fallen auch die vorstehend definierten gebrauchsfertigen Produkte und Lebensmittel (verzehrfertig oder als Lebensmittel-Halbfertigware). Es versteht sich, dass die im Rahmen des vorliegenden Textes verwendeten Bezeichnungen für bestimmte Artikelgruppen und Zubereitungen teilweise Erzeugnisse umfassen, welche unter mehrere Bezeichnungen fallen können. So gibt es beispielsweise der Ernährung dienende Zubereitungen, die gleichzeitig dem Genuss dienen, wobei es sich insbesondere um Lebensmittel handeln kann. Gemeinsam ist allen Bezeichnungen jedoch, dass sie Artikel (Produkte) betreffen, welche in die menschliche Mundhöhle eingebracht werden sollen, um dort einen Geruch- oder Geschmackseindruck zu verursachen.

In erfindungsgemäßen Riech- und/oder Aromastoffkompositionen, die (a) 4-Methyl-delta-hexalacton sowie (b) eine, mehrere oder sämtliche Verbindungen ausgewählt aus der Gruppe bestehend aus (i) Menthol, (ii) (-)-Carvon, (iii) Menthon, (iv) Isomenthon umfassen, vermag das erfindungsgemäß einzusetzende 4-Methyl-delta-hexalacton bereits in geringen Dosierungen das Gesamtbild der Riech- bzw. Aromastoffkomposition geruchlich bzw. geschmacklich abzurunden und insbesondere der Komposition mehr Authentizität und Natürlichkeit zu verleihen.

Im Falle eines rekonstituierten (rekombinierten, nachgestellten) d.h. nichtnatürlichen Pfefferminzöls (welches signifikante Mengen an Menthol enthält) bzw. Spearmintöls (welches signifikante Mengen an (-)-Carvon enthält) wird durch den Zusatz von 4-Methyl-delta-hexalacton der Formel A, insbesondere in den oben angegebenen Mengen, die Natürlichkeit und Authentizität jeweils deutlich erhöht, insbesondere wegen der durch das 4-Methyl-delta-hexalacton vermittelten Noten Heu und Cumarin, welche in natürlichem Pfefferminzöl und Spearmintöl ebenfalls vorhanden sind.

Durch Einarbeitung der Verbindung (c) Nonenolid (2-Nonen-4-olid; 4-Hydroxy-2-nonensäurelacton; IUPAC-Name: 5-Pentyl-5H-furan-2-on, 5-Pentyl-2(5H)-furanon) der Formel C: 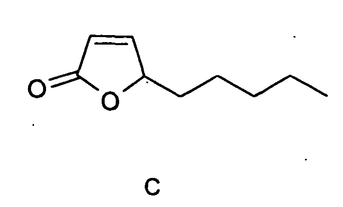 in einen erfindungsgemäßen parfümierten oder aromatisierten Artikel, insbesondere eine erfindungsgemäße Riech- oder Aromastoffkomposition, kommen insbesondere die durch das erfindungsgemäß einzusetzende 4-Methyl-delta-hexalacton der Formel A vermittelten, modifizierten und/oder verstärkten Noten Heu und Cumarin intensiver zur Geltung, so dass hinsichtlich dieser Noten eine noch größere Natürlichkeit und Authentizität erreicht werden kann, insbesondere bei der Herstellung rekonstituierter (rekombinierter, nachgestellter), d.h. nicht-natürlicher Pfefferminz- und/oder Spearmintöle bzw. -aromen.

Das Nonenolid der Formel C kann dabei als (R)-konfiguriertes, (S)-konfiguriertes oder beliebiges Gemisch der Enantiomeren, insbesondere als Racemat, vorliegen.

Dabei ist zu erwähnen, dass Nonenolid der Formel C in der Kombination mit 4-Methyl-delta-hexalacton der Formel A eine stärkere cumarinige und eine schwächer ausgeprägte heuartige Note bewirkt. Die Kombination der Verbindungen der Formeln A und C ermöglicht dadurch eine Maßschneiderung der Noten Heu und Cumarin, so dass auch ein sehr ausgeglichenes sensorisches Profil dieser beiden Noten erreicht werden kann.

Nonenolid der Formel C ist bekannt und kann beispielsweise hergestellt werden gemäß J. Food Science 1978, 43, 1248-1252 (racemische Synthese) oder J. Org. Chem. 1995, 60, 5628-5633 (enantioselektive Synthese).

In US 3,767,427 ist ausgeführt, dass 4-Hydroxy-2-nonensäurelacton (Nonenolid der Formel C) per se einen starken Geruch von süßer Kokosnuss und Frittiertem aufweist. US 3,767,427 beschreibt (im Wesentlichen fettfreie) Lebensmittel enthaltend eine Menge an Nonenolid im Bereich von 0,1 - 20 ppm, wodurch dem Lebensmittel eine fettige, frittierte Geruchs- und Geschmacksnote vermittelt wird, insbesondere in Kombination mit ungesättigten delta-Lactonen mit 7 bis 12 Kohlenstoffatomen oder 2,4-Decadienal. Auf diese Weise kann Produkten wie gebackenen Kartoffelchips oder Margarine ein fettiger, frittierter Geruchs- und Geschmackseindruck vermittelt werden.

Gemäß J. Food Science 1978, 43, 1248-1252 wurde Baumwollsamenöl mit 2,5 ppm Nonenolid versetzt und durch ein Panel organoleptisch bewertet, wobei nussige, frittierte Noten festgestellt wurden, manche Panellisten gaben auch Butter-ähnliche Noten an.

In Agr. Biol. Chem. 1970, 34, 1745-1747 werden ungesättigte gamma-Lactone (wie Nonenolid eines ist) im Großen und Ganzen als fettig, grasig und süßlich beschrieben.

In J. Agric. Food Chem. 1997, 45, 1329-1332 wird über die Ergebnisse der Aromaextrakt-Verdünnungsanalyse von gekochten Huitlacoche-Pilzen berichtet. Mittels GC/Olfaktometrie wurde 2-Nonensäure-gamma-lacton (Nonenolid der Formel C) identifiziert, welches geruchlich mit "Kokosnuss" beschrieben wurde.

In Biosci. Biotech. Biochem. 1993, 57, 79-81 wurde eine Fraktion von diversen Lactonen aus einem flüchtigen Kondensat aus halbfermentiertem Pouchong-Tee gewonnen. Die Autoren vermuten, dass diese Lactonfraktion, welche unter anderem als Spurenkomponente Nonenolid enthielt, zu dem charakteristischen Geruch des Pouchong-Tees beiträgt.

Bei der oxidativen Zersetzung von (E,E)-2,4-Decadienal in wässrigem Medium bei einem pH-Wert von 6,5 wurde gemäß J. Agric. Food Chem. 1993, 41, 2385-2390 ein Produktgemisch erhalten, das unter anderem 1,5 % Nonenolid enthielt.

Das dem erfindungsgemäß einzusetzenden Nonenolid der Formel C strukturell recht ähnliche gesättigte gamma-Nonalacton ist als nach Kokosnuss riechender bzw. schmeckender Stoff bekannt (vgl. K. Bauer et al., Common Fragrance and Flavor Materials, 4th Edition, Wiley-VCH, Weinheim 2001 oder auch Tobacco Science 1976, 20, 125-133).

Einige der in den oben bereits genannten Dokumenten Planta Medica 1985, 533-534 und Tobacco Science 1976, 20, 125-133 jeweils untersuchten Extrakte oder Fraktionen enthielten sowohl das gesättigte gamma-Nonalacton als auch 4-Methyl-delta-hexalacton der Formel A, jedoch nicht die erfindungsgemäß vorzugsweise einzusetzende Verbindung der Formel C.

Zusammenfassend besitzen daher die folgenden erfindungsgemäßen Riech- oder Aromastoffkompositionen (Mischungen) eine überraschende geruchliche und/oder geschmackliche Qualität:
- Riech- oder Aromastoffmischungen (= Kompositionen) umfassend 4-Methyl-delta-hexalacton und einen oder mehrere weitere Riech- oder Aromastoffe, wobei der Komposition durch das 4-Methyl-delta-hexalacton ein, mehrere oder sämtliche Geruchs- oder Geschmachseindrücke Heu, Cumarin, Lacton und/oder Mundfülle vermittelt, modifiziert und/oder verstärkt wird;
- Riech- oder Aromastoffmischungen (= Kompositionen) umfassend 4-Methyl-delta-hexalacton und eine, mehrere oder sämtliche Verbindungen der Gruppe (b) (i) Menthol und/oder (ii) (-)-Carvon und/oder (iii) Menthon und/oder (iv) Isomenthon sowie einen oder mehrere weitere Riech- oder Aromastoffe.

Ferner ist festzuhalten, dass insbesondere Riech- oder Aromastoffkompositionen sowie entsprechende Artikel umfassend
(a) 4-Methyl-delta-hexalacton,
(b) (i) I-Menthol und/oder (ii) (-)-Carvon
   sowie gegebenenfalls
(c) Nonenolid
   ganz besonders bevorzugt sind, da solche Kombinationen eine hervorragende Basis für die Herstellung rekonstituierter, (d.h. nicht-natürlicher) Pfefferminz- und Spearmintaromen sowie Pfefferminzöle und Spearmintöle darstellen.

Der erfindungsgemäßen Verwendung von 4-Methyl-delta-hexalacton entspricht ein erfindungsgemäßes Verfahren zum Vermitteln, Modifizieren und/oder Verstärken einer oder mehrerer Geruchs- oder Geschmackseindrücke aus der Gruppe Heu, Cumarin, Lacton und Mundfülle, wobei 4-Methyl-delta-hexalacton oder eine erfindungsgemäße 4-Methyl-delta-hexalacton umfassende Riech- oder Aromastoffkomposition (vorzugsweise in einer der oben als bevorzugt bezeichneten Ausgestaltungen) mit einem Erzeugnis in Kontakt gebracht oder gemischt wird.

Die sensorischen Eigenschaften, stofflichen Eigenschaften (wie Löslichkeit in gängigen Lösungsmitteln) sowie die Kompatibilität mit gängigen weiteren Bestandteilen von Riech- oder Aromastoffkompositionen unterstreichen die besondere Eignung des 4-Methyl-delta-hexalacton für die genannten Einsatzzwecke.

Die Erfindung betrifft auch ein Verfahren zur Herstellung einer Riech- oder Aromastoffkomposition, mit folgendem Schritt: Vermischen von 4-Methyl-delta-hexalacton mit üblichen Bestandteilen einer Riech- oder Aromastoffkomposition, wobei das 4-Methyl-delta-hexalacton in einer Menge eingesetzt wird, die ausreicht, um in der Riech- oder Aromastoffkomposition einen oder mehrere Geruchs- oder Geschmackseindrücke aus der Gruppe bestehend aus Heu, Cumarin, Lacton und Mundfülle zu vermitteln, zu modifizieren und/oder zu verstärken. Vorzugsweise ist die besagte Riech- oder Aromastoffkomposition dabei eine erfindungsgemäße Riech- oder Aromastoffkomposition. In diesem Fall wird (a) 4-Methyl-delta-hexalacton mit (b) einer, mehreren oder sämtlichen Verbindungen ausgewählt aus der Gruppe bestehend aus (i) Menthol, (ii) (-)-Carvon, (iii) Menthon und (iv) Isomenthon sowie vorzugsweise (c) Nonenolid und/oder (d) Menthylacetat sowie gegebenenfalls weiteren (üblichen) Bestandteilen einer Riech- oder Aromastoffkomposition vermischt.

Zutaten, mit denen 4-Methyl-delta-hexalacton der Formel A (in einer der genannten Formen) kombiniert werden kann, sind beispielsweise:
Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, alpha-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Einzelne zur Verwendung im Rahmen der vorliegenden Erfindung bevorzugte Kühlwirkstoffe zur Einarbeitung in erfindungsgemäße Riech- und Aromastoffkompositionen bzw. Artikel sind nachfolgend aufgelistet. Der Fachmann kann die nachfolgende Liste um eine Vielzahl weiterer Kühlwirkstoffe ergänzen; die aufgelisteten Kühlwirkstoffe können auch in Kombination miteinander eingesetzt werden: Menthonglycerinacetal (Handelsname: Frescolat^{®}MGA), Menthyllactat (Handelsname: Frescolat^{®}ML, vorzugsweise handelt es sich bei Menthyllactat um I-Menthyllactat, insbesondere I-Menthyl-I-lactat), substituierte Menthyl-3-carbonsäureamide (z.B. Menthyl-3-carbonsäure-N-ethylamid, auch als WS-3 bekannt), 2-Isopropyl-N-2,3-trimethylbutanamid (auch als WS-23 bekannt), substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.

Besonders bevorzugte Kühlwirkstoffe sind: Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), substituierte Menthyl-3-carbonsäureamide (z.B. Menthyl-3-carbonsäure-N-ethylamid), 2-Isopropyl-N-2,3-trimethylbutanamid, 3-Menthoxypropan-1,2-diol, 2-Hydroxyethylmenthylcarbonat, 2-Hydroxypropylmenthylcarbonat, Isopulegol und Monomenthylsuccinat.

Wie vorstehend bereits ausgeführt, ist es vorteilhaft, in erfindungsgemäßen Riech- oder Aromastoffkompositionen einige oder mehrere übliche Riech- oder Aromastoffe einzusetzen. Auf diese Weise lassen sich besonders interessante und natürliche neue und originelle Geruchs- oder Geschmacksnoten kreieren. Riech- und Aromastoffe, die zur Kombination vorteilhafterweise geeignet sind, finden sich z.B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001. Im einzelnen seien genannt:
Extrakte aus natürlichen Rohstoffen wie Etherische Öle, Concretes, Absolues, Resine, Resinoide, Balsame, Tinkturen wie z. B.
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-ÖI; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-ÖI; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-ÖI; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. 8-Mercaptomenthan-3-on; Campher; Fenchon; alpha-lonon; beta-lonon; alpha-n-Methylionon; beta-n-Methylionon; alpha-Isomethylionon; beta-Isomethylionon; alpha-lron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. 1,8-Cineol (Eucalyptol); Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.
Erfindungsgemäße Riech- oder Aromastoffkompositionen, die 4-Methyl-delta-hexalacton enthalten, können in flüssiger Form, unverdünnt oder mit einem Lösungsmittel verdünnt vorliegen und zur Parfümierung oder Aromatisierung eingesetzt werden. Geeignete Lösungsmittel hierfür sind z.B. Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin usw.

Des weiteren können erfindungsgemäße Riech- oder Aromastoffkompositionen, die 4-Methyl-delta-hexalacton enthalten, an einem Trägerstoff adsorbiert sein, der sowohl für eine feine Verteilung der Riech- oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff stellt einen beispielhaften erfindungsgemäßen Artikel dar.

Erfindungsgemäße Riech- oder Aromastoffkompositionen, die 4-Methyl-delta-hexalacton enthalten, können auch mikroverkapselt, sprühgetrocknet, als Einschluss-Komplexe oder als Extrusions-Produkte (d. h. erfindungsgemäße Artikel) vorliegen und in dieser Form z. B. einem zu parfümierenden oder aromatisierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Die Mikroverkapselung der erfindungsgemäßen Riech- oder Aromastoffkompositionen zu erfindungsgemäßen Artikeln kann beispielsweise durch das sogenannte Koazervationsverfahren (nahtlose Kapseln, basierend auf Gelatine und/oder Alginat), mit Hilfe von Kapselmaterialien z.B. aus polyurethanartigen Stoffen oder Weichgelatine, erfolgen. Die sprühgetrockneten Riech- oder Aromastoffkompositionen können beispielsweise durch Sprühtrocknung einer die Riech- oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riech- oder Aromastoffkomposition und Cyclodextrinen oder Harnstoffderivaten in ein geeignetes Lösungsmittel, z.B. Wasser, hergestellt werden. Extrusions-Produkte können durch Verschmelzen der Riech- oder Aromastoffkompositionen mit einem geeigneten wachsartigen Stoff und durch Extrusion mit nachfolgender Erstarrung, ggf. in einem geeigneten Lösungsmittel, z.B. Isopropanol, erhalten werden.

Riechstoffkompositionen, die 4-Methyl-delta-hexalacton enthalten, können in konzentrierter Form, in Lösungen oder in oben beschriebener modifizierter Form verwendet werden für die Herstellung von erfindungsgemäßen parfümierten Artikeln wie z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom ÖI-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Das erfindungsgemäß einzusetzende 4-Methyl-delta-hexalacton kann in aromatisierte oder zu aromatisierende Artikel eingearbeitet werden, insbesondere in der Ernährung, der Mundpflege oder dem Genuss dienende Zubereitungen.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, (fruchthaltige) Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäßen Verbindung sind diese Zubereitungen erfindungsgemäße Zubereitungen (als Beispiel erfindungsgemäßer Artikel). Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Bevorzugte zum Verzehr geeignete Produkte sind beispielsweise Backwaren (Kekse, Kuchen, Muffins, Waffeln, Backmischungen), Zuckerwaren (Hartkaramellen, Weichkaramellen, Kaubonbons, Komprimate, Dragees, Zuckerperlen, Zuckerfüllungen), Milchprodukte (Joghurts, Puddings, Eiscreme), Schokoladenwaren (weiße, helle oder dunkle Schokolade, Schokoladenriegel), Fettmassen (Backwarenfüllungen wie z.B. Keksfüllungen, Schokoladenfettfüllungen, Riegelfettfüllungen), Kaugummis (zuckerfrei, zuckerhaltig, Streifen, Komprimate, Dragees), Snacks und Snackmischungen, wasserlösliche Pulverprodukte, Aufstreumischungen (Toppings), vergleiche auch die obigen Ausführungen zu bevorzugten (gebrauchsfertigen) erfindungsgemäßen Zubereitungen.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,9 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,9 Gew.-%, vorzugsweise 5 bis 80 Gew.-%, bezogen auf das Gesamtgewicht der Zubereitung, aufweisen.

Die erfindungsgemäßen Zubereitungen (als Beispiele erfindungsgemäßer Artikel), enthaltend eine erfindungsgemäße Riech- oder Aromastoffmischung, werden gemäß einer bevorzugten Ausgestaltung hergestellt, indem das 4-Methyl-delta-hexalacton als Substanz, als Lösung (z.B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden. Als sonstige Aromen oder Aromastoffe werden dabei insbesondere - wie vorstehend ausgeführt - eine, mehrere oder sämtliche Verbindungen ausgewählt aus der Gruppe bestehend aus (i) Menthol, (ii) (-)-Carvon, (iii) Menthon und (iv) Isomenthon sowie vorzugsweise (c) Nonenolid und/oder (d) Menthylacetat eingesetzt.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen das erfindungsgemäß einzusetzende 4-Methyl-delta-hexalacton bzw. eine erfindungsgemäße Riech- oder Aromastoffkomposition und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung wird das 4-Methyl-delta-hexalacton bzw. eine erfindungsgemäße Riech- oder Aromastoffkomposition umfassend 4-Methyl-delta-hexalacton zunächst mit einem oder mehreren geeigneten Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt ist, dass 4 -Methyl-delta-hexalacton bzw. eine erfindungsgemäße Riech- oder Aromastoffkomposition verzögert von der Matrix freigegeben wird, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2 -Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für der Mundpflege dienende erfindungsgemäße Zubereitungen), die erfindungsgemäß einzusetzende 4-Methyl-delta-hexalacton bzw. eine erfindungsgemäße 4-Methyl-delta-hexalacton umfassende Riech- oder Aromastoffkompositionen enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Mentholderivate (z.B. L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende erfindungsgemäße Zubereitungen), welche das erfindungsgemäß einzusetzende 4-Methyl-delta-hexalacton bzw. eine erfindungsgemäße 4-Methyl-delta-hexalacton enthaltende Riech- oder Aromastoffkomposition enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Im Stand der Technik sind zahlreiche unterschiedliche Kaugummibasen bekannt, wobei zwischen sogenannten "chewing gum" - oder um "bubble gum" - Basen zu unterscheiden ist, wobei letztere weicher sind, damit sich damit auch Kaugummiblasen bilden lassen. Gängige Kaugummibasen umfassen neben traditionell eingesetzten natürlichen Harzen oder dem Naturlatex Chicle heute zumeist Elastom ere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerin-monostearat.

Nachfolgend wird die Erfindung anhand von Beispielen näher erläutert. Sofern nicht anders angegeben, beziehen sich alle Angaben auf das Gewicht.

### Beispiel 1: Darstellung von 4-Methyl-delta-hexalacton

Das folgende Schema verdeutlicht die hier genutzte Reaktionssequenz: 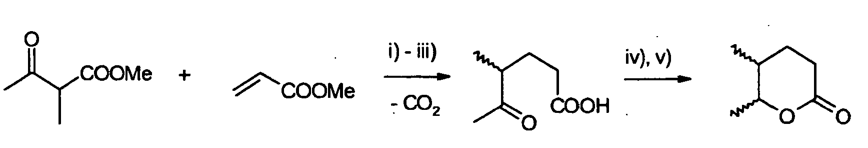
i) NaOMe / MeOH, ii) KOH / H₂O; iii) H₂SO₄ / H₂O; iv) NaBH₄, v) H₂SO₄

Zu 402 g (2,8 mol) 2-Methylacetessigsäuremethylester (98%ig) wurde unter kräftigem Rühren bei Raumtemperatur (ca. 20°C) beginnend eine Lösung von 7,5 g (0,14 mol) Natriummethylat (95%ig) in 30 mL wasserfreiem Methanol getropft; anschließend wurden hierzu bei 30°C (Innentemperatur) unter Rühren 225 g (2,6 mol) Acrylsäuremethylester getropft. Das erhaltene Reaktionsgemisch wurde 15 h bei Raumtemperatur gerührt, dann zu einer Lösung von 360 g (5,5 mol) Kaliumhydroxid (85%ig) in 1000 mL Wasser und 700 mL Methanol getropft, 12 h bei Raumtemperatur gerührt und anschließend mit einer Lösung von 300 g konzentrierter Schwefelsäure (96%ig) in 400 mL Wasser unter Kühlung im Eisbad angesäuert. Das resultierende Gemisch wurde im Vakuum (20 mbar / 40°C Badtemperatur) eingeengt, mit 1500 mL Wasser verdünnt und dann viermal mit je 400 mL Ether extrahiert. Die vereinigten Etherextrakte wurden im Vakuum (20 mbar / 40°C Badtemp.) eingeengt. Der hierbei verbliebene Rückstand (363 g) wurde nun bei Raumtemperatur zu einer Lösung von 170 g (2,58 mol) Kaliumhydroxid (85%ig) in 1400 mL Wasser getropft und anschließend eine Lösung von 2 g 50%iger wässeriger Natronlauge und 24 g (0,64 mol) Natriumboranat in 100 mL Wasser bei Raumtemperatur zugetropft, das resultierende Reaktionsgemisch 12 h bei Raumtemperatur gerührt, mit 90 g (0,9 mol) konzentrierter Schwefelsäure (96%ig) angesäuert und viermal mit je 400 mL Ether extrahiert. Die vereinigten Etherextrakte wurden im Vakuum (20 mbar, 40°C Badtemp.) eingeengt und der erhaltene Rückstand anschließend im Vakuum über eine 50 cm Vigreux-Kolonne fraktionierend destilliert. Bei einer Kopftemperatur von 59 - 60°C / 0,76 mbar wurden 131 g des Produktes der Formel A (nach GC ein 1 : 1-Gemisch beider syn-und anti-Diastereomere) als farblose Flüssigkeit (Reinheit nach GC: 98,6%, Summe beider Diastereomere) erhalten.

1 H-NMR (400 MHz/CDCl₃/ TMS): 0,98 (d, 4- CH₃, J = 6,95 Hz, Diastereomer 1), 1,01 (d, 4-CH₃, J = 6,47 Hz, Diastereomer 2), 1,30 (d, CH₃, J = 6,66 Hz, Diastereomer 1), 1,37 (d, CH₃, J = 6,26 Hz, Diastereomer 2), 1,50 - 1,74 (m, 3-CH₂, beide Diastereomere), 1,88 - 2,10 (m, 4-CH, beide Diastereomere), 2,46 - 2,66 (m, 2-CH₂, beide Diastereomere), 4,03 - 4,09 (m, 5-CH, Diastereomer 1), 4,52 - 4,56 ppm (m, 5-CH, Diastereomer 2).

13C-NMR (75 MHz/CDCl₃/TMS): 13,06 (q, 4-CH₃, Diastereomer 1), 17,30 (q, CH₃, Diastereomer 1 und 4-CH₃, Diastereomer 2), 19,97 (q, CH₃, Diastereomer 2), 25,37 (t, 3-CH₂, Diastereomer 1), 27,05 (t, 2-CH₂, Diastereomer 1), 27,78 (t, 3-CH₂, Diastereomer 2), 29,62 (t, 2-CH₂, Diastereomer 2), 30,37 (d, 4-CH, Diastereomer 1), 34,59 (d, 4-CH, Diastereomer 2), 79,09 (d, 5-CH, Diastereomer 1), 82,43 (d, 5-CH, Diastereomer 2), 171,79 ppm (s, C=O, Diastereomer 1 und 2).

FT-IR: 1097 (m), 1250 (m), 1257 (m), 1730 (s), 2879 (m), 2937 (m), 2974 1/cm (m).

MS (70 MeV) m/z = 128 (2,4%, M+), 84 (36%, M - CO2), 56 (100%), 43 (28%), 42 (37%), 41 (26%).

### Beispiel 2: Aromastoffkompositionen (synthetische Pfefferminzöle)

| | **Aroma P** | **Aroma A1** | **Aroma A2** |
|---|---|---|---|
| | Gewichtsteile | Gewichtsteile | Gewichtsteile |
| Isobutyraldehyd | 0,5 | 0,5 | 0,5 |
| 3-Octanol | 0,5 | 0,5 | 0,5 |
| Dimethylsulfid | 0,5 | 0,5 | 0,5 |
| Trans-2-Hexenal | 1,0 | 1,0 | 1,0 |
| Cis-3-Hexenol | 1,0 | 1,0 | 1,0 |
| 4-Terpineol, natürlich | 1,0 | 1,0 | 1,0 |
| Isopulegol | 1,0 | 1,0 | 1,0 |
| Piperiton, natürlich, aus Eucalyptus | 2,0 | 2,0 | 2,0 |
| Linalool | 3,0 | 3,0 | 3,0 |
| 8-Ocimenylactetat, 10% in Triacetin | 5,0 | 5,0 | 5,0 |
| Isoamylalkohol | 10,0 | 10,0 | 10,0 |
| Isovaleraldehyd | 10,0 | 10,0 | 10,0 |
| Alpha-Pinen | 25,0 | 25,0 | 25,0 |
| Beta-Pinen, natürlich | 25,0 | 25,0 | 25,0 |
| Neomenthol, racemisch | 40,0 | 40,0 | 40,0 |
| Eucalyptol (1,8-Cineol), natürlich | 50,0 | 50,0 | 50,0 |
| L-Menthylacetat der Formel D | 70,0 | 70,0 | 70,0 |
| L-Menthon | 220,0 | 220,0 | 220,0 |
| D-Isomenthon | 50,0 | 50,0 | 50,0 |
| L-Menthol | 482,5 | 482,5 | 482,5 |
| 4-Methyl-delta-hexalacton der Formel A, aus Beispiel 1 | - | 1,0 | 1,0 |
| Nonenolid der Formel C, racemisch | - | - | 1,0 |
| Total: | 998,0 | 999,0 | 1000,0 |

Nach Meinung der Flavoristen bekommt das Aroma P (nicht erfindungsgemäß), eine nicht-natürliche Nachstellung eines Pfefferminzöls, durch den Zusatz von 1 Gewichtsteil des 4-Methyl-delta-hexalactons (Aroma A1) eine Note von Heu und Cumarin. 4-Methyl-delta-hexalacton fügt sich gut in die Komposition ein, verleiht der Komposition mehr Natürlichkeit und Authentizität und rundet diese ab. Diese Noten bzw. Effekte werden durch den weiteren Zusatz von 1 Gewichtsteil Nonenolid (Aroma A2) noch verstärkt.

### Formulierungsbeispiele

### Beispiel F1: Gel-Zahncreme mit Wirksamkeit gegen Mundgeruch

| | I (Gew.-%) | II (Gew.-%) | III (Gew.-%) |
|---|---|---|---|
| Na-carboxymethylcellulose | 0,40 | 0,40 | 0,40 |
| Sorbitol 70 %, in Wasser | 72,00 | 72,00 | 72,00 |
| Polyethylenglykol (PEG) 1500 | 3,00 | 3,00 | 3,00 |
| Na-saccharinat | 0,07 | 0,07 | 0,07 |
| Na-fluorid | 0,24 | 0,24 | 0,24 |
| p-Hydroxybenzoesäure (PHB)-Ethylester | 0,15 | 0,15 | 0,15 |
| Aroma A1 aus Beispiel 2 | 0,50 | 0,80 | 1,50 |
| Abrasivkieselsäure | 11,00 | 11,00 | 11,00 |
| Verdickungskieselsäure | 6,00 | 6,00 | 6,00 |
| Natriumdodecylsulfat (SDS) | 1,40 | 1,40 | 1,40 |
| Wasser dest. | Ad 100,00 | Ad 100,00 | Ad 100,00 |

### Beispiel F2: Zahncreme gegen Plaque

| | I (Gew.-%) | II (Gew.-%) | III (Gew.-%) |
|---|---|---|---|
| Na-carboxymethylcellulose | 1,00 | 1,00 | 1,00 |
| Glycerin | 12,50 | 12,50 | 12,50 |
| Sorbitol 70 %, in Wasser | 29,00 | 29,00 | 29,00 |
| Na-saccharinat | 0,20 | 0,20 | 0,20 |
| Na-fluorid | 0,22 | 0,22 | 0,22 |
| Azacycloheptan-2,2-diphosphosäure, Di-natriumsalz | 1,00 | 1,00 | 1,00 |
| Bromchlorophen | 0,10 | 0,10 | 0,10 |
| Aroma A2 aus Beispiel 2 | 0,15 | 0,90 | 1,20 |
| Abrasivkieselsäure | 15,00 | 15,00 | 15,00 |
| Verdickungskieselsäure | 5,00 | 5,00 | 5,00 |
| Natriumdodecylsulfat (SDS) | 1,50 | 1,50 | 1,50 |
| Wasser dest. | Ad 100,00 | Ad 100,00 | Ad 100,00 |

### Beispiel F3: Zahncreme gegen Plaque

| | I (Gew.-%) | II (Gew.-%) | III (Gew.-%) |
|---|---|---|---|
| Carragenan | 0,90 | 0,90 | 0,90 |
| Glycerin | 15,00 | 15,00 | 15,00 |
| Sorbitol 70 %, in Wasser | 25,00 | 25,00 | 25,00 |
| PEG 1000 | 3,00 | 3,00 | 3,00 |
| Na-fluorid | 0,24 | 0,24 | 0,24 |
| Tetrakalium-diphosphat | 4,50 | 4,50 | 4,50 |
| Tetranatrium-diphosphat | 1,50 | 1,50 | 1,50 |
| Na-saccharinat | 0,40 | 0,40 | 0,40 |
| Fällungskieselsäure | 20,00 | 20,00 | 20,00 |
| Titandioxid | 1,00 | 1,00 | 1,00 |
| PHB-Methylester | 0,10 | 0,10 | 0,10 |
| Anethol-Eucalyptol - Aroma | 0,10 | 0,25 | 0,20 |
| Aroma A1 aus Beispiel 2 | 0,50 | 0,70 | 1,00 |
| Natriumdodecylsulfat | 1,30 | 1,30 | 1,30 |
| Wasser dest. | Ad 100,00 | Ad 100,00 | Ad 100,00 |

### Beispiel F4: Zahncreme gegen empfindliche Zähne

| | I (Gew.-%) | II (Gew.-%) | III (Gew.-%) |
|---|---|---|---|
| Na-carboxymethylcellulose | 0,70 | 0,70 | 0,70 |
| Xanthan Gum | 0,50 | 0,50 | 0,50 |
| Glycerin | 15,00 | 15,00 | 15,00 |
| Sorbitol 70 %, in Wasser | 12,00 | 12,00 | 12,00 |
| K-nitrat | 5,00 | 5,00 | 5,00 |
| Na-monofluorphosphat | 0,80 | 0,80 | 0,80 |
| PHB-Methylester | 0,15 | 0,15 | 0,15 |
| PHB-Propylester | 0,05 | 0,05 | 0,05 |
| Na-saccharinat | 0,20 | 0,20 | 0,20 |
| Wintergrün - Aroma (enthält Methylsalicylat) | 0,05 | 0,15 | 0,10 |
| Aroma A1 aus Beispiel 2 | 0,25 | 0,65 | 1,10 |
| Ca-carbonat | 35,00 | 35,00 | 35,00 |
| Siliciumdioxid | 1,00 | 1,00 | 1,00 |
| Natriumdodecylsulfat (SDS) | 1,50 | 1,50 | 1,50 |
| Wasser dest. | Ad 100,00 | Ad 100,00 | Ad 100,00 |

### Beispiel F5: Zahncreme gegen empfindliche Zähne

| | I (Gew.-%) | II (Gew.-%) | III (Gew.-%) |
|---|---|---|---|
| Hydroxyethylcellulose | 1,40 | 1,40 | 1,40 |
| Guar Gum | 0,60 | 0,60 | 0,60 |
| Glycerin | 18,00 | 18,00 | 18,00 |
| Sorbitol 70 %, in Wasser | 12,00 | 12,00 | 12,00 |
| Na-Saccharinat | 0,35 | 0,35 | 0,35 |
| Farbstoff | 0,01 | 0,01 | 0,01 |
| PHB-Methylester | 0,15 | 0,15 | 0,15 |
| PHB-Propylester | 0,04 | 0,04 | 0,04 |
| Sr-chlorid | 10,50 | 10,50 | 10,50 |
| Aroma A2 aus Beispiel 2 | 0,40 | 1,00 | 1,50 |
| Fällungskieselsäure | 15,00 | 15,00 | 15,00 |
| Siliciumdioxid | 1,60 | 1,60 | 1,60 |
| Natriumdodecylsulfat | 1,30 | 1,30 | 1,30 |
| Wasser dest. | Ad 100,00 | Ad 100,00 | Ad 100,00 |

### Beispiel F6: Gebrauchsfertiges Mundwasser mit Fluorid

| | I (Gew.-%) | II (Gew.-%) | III (Gew.-%) |
|---|---|---|---|
| Ethanol | 7,00 | 7,00 | 7,00 |
| Glycerin | 12,00 | 12,00 | 12,00 |
| Na-fluorid | 0,05 | 0,05 | 0,05 |
| Pluronic F-127^{®} (BASF, oberflächenaktive Substanz) | 1,40 | 1,40 | 1,40 |
| Na-phosphatpuffer pH 7,0 | 1,10 | 1,10 | 1,10 |
| Sorbinsäure | 0,20 | 0,20 | 0,20 |
| Na-saccharinat | 0,10 | 0,10 | 0,10 |
| Aroma A2 aus Beispiel 2 | 0,10 | 0,25 | 0,40 |
| Farbstoff | 0,01 | 0,01 | 0,01 |
| Wasser dest. | Ad 100,00 | Ad 100,00 | Ad 100,00 |

### Beispiel F7: Mundwasserkonzentrat

| | I (Gew.-%) | II (Gew.-%) | III (Gew.-%) |
|---|---|---|---|
| Ethanol, 95%ig | 80,00 | 80,00 | 80,00 |
| Na-cyclamat | 0,15 | 0,15 | 0,15 |
| Eucalyptol -Aroma (enthält natürliches Eucalyptol) | 1,00 | 1,00 | 1,00 |
| Farbstoff | 0,01 | 0,01 | 0,01 |
| Aroma A1 aus Beispiel 2 | 2,00 | 3,00 | 4,00 |
| Wasser dest. | Ad 100,00 | Ad 100,00 | Ad 100,00 |

### Beispiel F8: Zuckerhaltige Kaugummis

| | I (Gew.-%) | II (Gew.-%) | III (Gew.-%) |
|---|---|---|---|
| Kaugummibase | 21,00 | 21,00 | 21,00 |
| Glucose Sirup | 16,50 | 16,50 | 16,50 |
| Glycerin | 0,50 | 0,50 | 0,50 |
| Puderzucker | 60,45 | 60,40 | 60,30 |
| Verkapseltes Wintergrün-Aroma (enthält Methylsalicylat) | 1,20 | 1,00 | 0,70 |
| Aroma A2 aus Beispiel 2 | 0,35 | 0,60 | 1,00 |

### Beispiele F9a - 9d: Zuckerfreie Kaugummis

### Beispiel F9a: Non-stick Kaugummi

Die Kaugummibase K1 bestand aus 2,0% Butyl Rubber (Isobuten-Isopren-Copolymer, MW 400000), 6,0% Polyisobuten (MW = 43800), 43,5% Polyvinylacetat (MW = 12000), 31,5% Polyvinylacetat (MW = 47000), 6,75% Triacetin und 10,25% Calciumcarbonat. Die Herstellung der Kaugummibase K1 und der Kaugummis kann analog zu US 5,601,858 erfolgen.

| | I (Gew.-%) | II (Gew.-%) | III (Gew.-%) |
|---|---|---|---|
| Kaugummibase K1 | 26,00 | 26,00 | 26,00 |
| Triacetin | 0,25 | 0,25 | 0,25 |
| Lecithin | 0,50 | 0,50 | 0,50 |
| Sorbitol, kristallin | 40,90 | 40,60 | 40,50 |
| Mannitol | 15,30 | 15,20 | 15,10 |
| Glycerin | 12,10 | 12,00 | 11,80 |
| Aspartam | 0,17 | 0,17 | 0,17 |
| Verkapseltes Aspartam | 1,08 | 1,08 | 1,08 |
| Amorphes Silica | 1,00 | 1,00 | 1,00 |
| Baumwollsamenöl | 0,50 | 0,50 | 0,50 |
| Polyoxyethylen-sorbitan-monolaurat (E-432) | 1,00 | 1,00 | 1,00 |
| Verkapseltes Spearmint-Aroma (enthält I-Carvon) | 0,20 | 0,10 | 0,20 |
| Verkapseltes Wintergrün-Aroma (enthält Methylsalicylat) | - | 0,20 | - |
| Aroma A1 aus Beispiel 2 | 1,00 | 1,40 | 1,70 |
| l-Menthyl-l-lactat | 0,10 | - | 0,20 |

### Beispiel F9b: Bubble gum

Die Herstellung der Bubble Gums kann analog zu US 5,093,136 erfolgen.

| | I (Gew.-%) | II (Gew.-%) |
|---|---|---|
| Styrol-Butadien-Copolymer (SBR) | 19,50 | 17,50 |
| Polyisobuten | 8,00 | 8,00 |
| Sorbitol Pulver | 49,19 | 47,19 |
| Sorbitol, 70 %, in Wasser | 9,20 | 22,20 |
| Hydrierte Stärkehydrolysate (HSH) | 9,00 | - |
| Glycerin | 3,00 | 2,00 |
| Aspartam | 0,10 | 0,10 |
| Verkapseltes Aspartam | 0,50 | 0,50 |
| Grüner und blauer Farbstoff | 0,01 | 0,01 |
| Aroma A2 aus Beispiel 2 | 1,50 | 2,50 |

Die Kaugummis der Rezeptur (I) wurden als kompakte Kugeln, die der Rezeptur (II) als Hohlkugeln ausgeformt.

### Beispiel F9c:

Die Kaugummibase K2 bestand aus 28,5% Terpenharz, 33,9% Polyvinylacetat (MW = 14000), 16,25% hydriertes Pflanzenöl, 5,5% Mono- und Diglyceride, 0,5% Polyisobuten (MW 75000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis kann analog zu US 6,986,907 erfolgen.

| | I (Gew.-%) | II (Gew.-%) | III (Gew.-%) |
|---|---|---|---|
| Kaugummibase K2 | 25,30 | 27,30 | 26,30 |
| Sorbitol | 61,48 | 59,48 | 61,80 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,68 |
| Menthol, sprühgetrocknet | 0,50 | - | - |
| Kirscharoma, sprühgetrocknet | - | 1,20 | - |
| Aroma A1 aus Beispiel 2, sprühgetrocknet | 1,50 | 1,80 | - |
| Aroma A2 aus Beispiel 2 | 1,00 | - | 1,68 |

Die Kaugummis der Rezeptur (I) und (II) wurden als Streifen, die der Rezeptur (III) als Pellets ausgeformt.

### Beispiel F9d:

| | I (Gew.-%) | II (Gew.-%) | III (Gew.-%) |
|---|---|---|---|
| Kaugummibase | 30,00 | 30,00 | 30,00 |
| Sorbit, Pulver | 38,45 | 38,40 | 38,30 |
| Palatinit | 9,50 | 9,50 | 9,50 |
| Xylit | 2,00 | 2,00 | 2,00 |
| Mannit | 3,00 | 3,00 | 3,00 |
| Aspartam | 0,10 | 0,10 | 0,10 |
| Acesulfam K | 0,10 | 0,10 | 0,10 |
| Emulgum / Emulgator | 0,30 | 0,30 | 0,30 |
| Sorbitol 70 %, in Wasser | 14,00 | 14,00 | 14,00 |
| Glycerin | 1,00 | 1,00 | 1,00 |
| Anis-Zimt-Aroma | 1,10 | 0,60 | 0,50 |
| Aroma A1 aus Beispiel 2 | 0,45 | 0,80 | 1,00 |
| l-Menthyl-l-lactat | - | 0,20 | 0,10 |
| 2-Hydroxypropyl- menthylcarbonat | - | | 0,10 |

### Beispiel F10: Gelatinekapsel zum Direktverzehr

| | I (Gew.-%) | II (Gew.-%) | III (Gew.-%) |
|---|---|---|---|
| Gelatinehülle: | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| | | | |

| Kernzusammensetzung: | | | |
|---|---|---|---|
| Pflanzenöl-Triglycerid (Kokosöl - Fraktion) | 79,39 | 68,40 | 58,25 |
| Aroma A2 aus Beispiel 2 | 10,0 | 20,0 | 28,65 |
| Neotam und Aspartam | 0,01 | 0,05 | - |
| Sucralose | 0,22 | 0,30 | 0,70 |
| 2-Hydroxypropyl-menthylcarbonat | 0,33 | 0,20 | - |
| 2-Hydroxyethylmenthyl-carbonat | - | 0,20 | 1,00 |
| (1 R,3R,4S) Menthyl-3-carbonsäure-N-ethylamid (WS-3) | - | 0,55 | 0,50 |
| (-)-Menthonglycerinacetal-(Frescolat MGA) | - | 0,30 | 0,80 |
| Vanillin | 0,05 | - | 0,10 |

Die zum Direktverzehr geeignete Gelatinekapsel wurde gemäß WO 2004/050069 hergestellt und hatte einen Durchmesser von 5 mm, das Gewichtsverhältnis von Kernmaterial zu Hüllmaterial lag bei 90 : 10. Die Kapsel öffnete sich im Mund innerhalb von weniger als 10 Sekunden und löste sich vollständig innerhalb von weniger als 50 Sekunden auf.

### Beispiel F11: Kaubonbon mit erfindungsgemäßem Aroma

| | | |
|---|---|---|
| Wasser | | 7,8 % |
| Zucker | Raffinade C4 | 42,1 % |
| Glucose Sirup | Dextrose 40 | 37,3 % |
| gehärtetes Pflanzenfett | Schmelzpunkt 32-36°C | 6,6 % |
| Lecithin | Emulgator (Sojalecithin) | 0,3 % |
| Gelatine | Schweinegelatine | 0,8 % |
| Fondant | Typ - S30 | 4,9 % |
| Aroma A1 aus Beispiel 2 | | 0,2 % |

### Herstellungshinweise:

a) Gelatine mit Wasser (1,8 fache Menge der Gelatine) bei 70°C 2 Stunden quellen lassen;
b) Zucker, Sirup, Wasser, Fett und Lecithin auf 123°C kochen;
c) Gelatinelösung langsam mit dem Kochansatz vermischen;
d) Aroma aus Beispiel 2 und optional Farbe unterrühren;
e) resultierende Masse auf einem Kühltisch auf ca. 70°C temperieren, danach Fondant zugeben und auf einer Ziehmaschine ca. 3 Minuten belüften;
f) Kaubonbonmasse anschließend schneiden und verpacken.

Beim Verzehr der Kaubonbons wird ein kräftiger Pfefferminzgeschmack während des Kauens wahrgenommen.

### Beispiel F12: Komprimate mit erfindungsgemäßem Aroma

| | |
|---|---|
| Dextrose | 98,0 Gew.-% |
| Magnesium Stearat (Gleitmittel) | 0,9 Gew.-% |
| Citronensäure | 0,3 Gew.-% |
| Aroma A2 aus Beispiel 2 | 0,8 Gew.-% |

Herstellungshinweise: Alle Bestandteile mischen und in einer geeigneten Maschine zu Komprimaten verpressen.

### Beispiel F13: Götterspeise mit erfindungsgemäßem Aroma

| | | |
|---|---|---|
| Zucker | Saccharose | 81,9 Gew.-% |
| Stabilisator | Hamulsion GGF Hahn & Co., Lübeck | 12,0 Gew.-% |
| Citronensäure | gemahlen | 3,0 Gew.-% |
| Trinatriumcitrat | Gelierhilfe | 2,0 Gew.-% |
| grüne Farbe | | 0,05 Gew.-% |
| Aroma A1 aus Beispiel 2 | | 1,05 Gew.-% |

Herstellungshinweis: 41 g dieser Mischung in 250 mL kochendes Wasser einrühren und erkalten lassen.

### Beispiel F14: Extrudat mit erfindungsgemäßem Aroma

| | | |
|---|---|---|
| Glukosesirup, sprühgetrocknet (DE-Wert: 31-34) | Glucidex IT33W (Firma Roquette) | 62,0 % |
| Maltodextrin (DE-Wert: 17-20) | (Firma Cerestar) | 28,4 % |
| Emulgator Monomuls | Emulgator auf der Basis von gehärtetem Palmöl; Schmelzpunkt: 64°C, (Firma Grünau) | 1,8 % |
| Dextrosemonohydrat (DE-Wert: 99,5) | Dextrose, kristallwasserhaltig (Firma Cerestar) | 1,8% |
| Wasser | | 2,0 % |
| Aroma A2 aus Beispiel 2 | | 4,0 % |

Herstellungshinweis (siehe auch WO 03/092412):
Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten.

### Beispiel F15: Wirbelschichtgranulate mit erfindungsgemäßem Aroma

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: interner Schlauchfilter) wird eine Lösung bestehend aus 44 Gew.-% Wasser, 11 Gew.-% erfindungsgemäßem Aroma A1 aus Beispiel 2, 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Beispiel F16: Teebeutel mit schwarzem Tee bzw. Rooibos-Tee und Granulaten enthaltend erfindungsgemäßes Aroma

### Beispiel F16a:

800 g Rotbuschtee (Rooibos-Tee) und 33 g der Aromapartikel aus Beispiel F14 mit erfindungsgemäßem Aroma A2 aus Beispiel 2 wurden gemischt, portioniert und anschließend in Teebeutel abgefüllt.

### Beispiel F16b:

850 g Schwarztee Fannings wurden in einem 5 Liter Lödige Pflugscharmischer vorgelegt und 10 Sekunden vorgemischt und fluidisiert. Ohne Unterbrechung des Mischprozesses wurde mittels einer Ein- oder Zweistoffdüse 6 g eines feinen Neutralöl-Nebels (Medium-Chain-Triglyceride-Ölaerosol) auf die fluidisierten Teeblätter gesprüht. Dies dauerte etwa 60 Sekunden. Ohne Unterbrechung des Mischprozesses wurden anschließend 40 g der oben genannten eingefärbten Aromapartikel aus Beispiel F15 mit erfindungsgemäßem Aroma A1 aus Beispiel 2 zu der fluidisierten Mischung gegeben und die gesamte Mischung weitere 60 Sekunden gemischt. Die so erhaltene Mischung wurde anschließend portioniert und in Teebeutel abgefüllt.

### Beispiel F17: Kaugummidragees, zuckerfrei

### Q1: Bestandteile Kaugummi-Rohmasse

| | I (Gew.-%) | II (Gew.-%) | III (Gew.-%) |
|---|---|---|---|
| Kaugummibase | 37,00 | 37,00 | 37,00 |
| Sorbit, Pulver | 50,50 | 50,50 | 50,50 |
| Aspartam | 0,20 | 0,20 | 0,20 |
| Plasticizer (Emulgum) | 0,50 | 0,50 | 0,50 |
| Acesulfame K | 0,20 | 0,20 | 0,20 |
| Sorbit 70 %ig in Wasser | 5,00 | 5,00 | 5,00 |
| Glycerin | 4,00 | 4,00 | 4,00 |
| Aroma A1 aus Beispiel 2 | 1,20 | 1,40 | 1,60 |
| Pfefferminzöl-Aroma (Optamint^{®}, Symrise) | 0,40 | 0,20 | - |
| Menthol, kristallin | 1,00 | 0,50 | 0,10 |
| Menthol, sprühgetrocknet | - | 0,50 | 0,90 |

### Q2: Bestandteile Coating (Überzug)

(die angegebenen Gewichtsanteile beziehen sich auf die Gesamtmasse des auf die Kaugummikissen (Q1) aufgebrachten Coatings (Q2); die Gesamtmasse an Q2 betrug etwa 35% bezogen auf die Masse Q1)

| | I (Gew.-%) | II (Gew.-%) | III (Gew.-%) |
|---|---|---|---|
| **Mischung A** | | | |
| Isomalt | 0,20 | 0 | 0 |
| Sorbitol | 0 | 0,40 | 0 |
| Mannitol | 0 | 0 | 0,80 |
| I-Menthol, sprühgetrocknet | 0,20 | 0,60 | 1,00 |

| **Mischung B** | | | |
|---|---|---|---|
| Isomalt | 68,00 | 67,70 | 67,40 |
| Wasser | 26,7 | 26,6 | 26,5 |
| Gummi Arabicum 40%ig in Wasser (dieser Anteil beinhaltet die für die Gummierung verwendete Menge) | 2,50 | 2,50 | 2,50 |
| Acesulfame K | 0,05 | 0,05 | 0,05 |
| Aspartam | 0,05 | 0,05 | 0,05 |
| Titandioxid | 1,50 | 1,50 | 1,50 |

| **Bestandteil C** | | | |
|---|---|---|---|
| Aroma A2 aus Beispiel 2 | 0,80 | 0,60 | 0,20 |

Alle Bestandteile der Kaugummi-Rohmasse (Q1) wurden gemischt, in Kaugummistränge geprägt und anschließend zu einzelnen Kaugummikissen geformt. Die Kaugummikissen wurden danach in einer rotierenden Dragiertrommel mit einer 40 Gew.-%igen Gummi Arabicum Lösung benetzt (gummiert). Die gummierten Kaugummikissen wurden anschließend in einer rotierenden Dragiertrommel mit der pulvrigen Mischung A überzogen, welche im Wesentlichen bestand aus sprühgetrocknetem I-Menthol und mindestens einem Zuckeraustauschstoff (meist gewählt aus Isomalt, Sorbit, Xylit, Maltit und/oder Mannit, gegebenenfalls kann zusätzlich pulvriges Gummi Arabicum verwendet werden). Nach ausreichender Trocknung mit kalter Luft wurden die so beschichteten Kaugummikissen über Nacht getrocknet. Zum weiteren Aufbringen des Coatings auf die getrockneten, beschichteten Kaugummikissen mit der Coatinglösung B wurden zunächst 15 Schichten mittels Dragierung aufgetragen, in der 16. Schicht wird ein Gemisch aus Bestandteil C und Mischung B aufgebracht. Es wurden danach weitere Schichten unter Verwendung der Mischung B aufgetragen, bis das Gesamtgewicht des Coatings (Q2) etwa 35 Gew.-% des Gewichts der ursprünglichen Kaugummikissen (Q1) betrug. Um den Kaugummidragees Glanz zu verleihen erfolgte eine abschließende Behandlung mit einem Glanzmittel, welches aus einer Mischung gleicher Gewichtsanteile von Carnauba- und Bienenwachs bestand. Die gebrauchsfertigen Kaugummidragees rufen beim Kauen im Mund einen sehr klaren, strahlenden, frischen mentholigen und natürlichen Pfefferminzgeschmack hervor.

### Beispiel F18: Sprühgetrocknetes erfindungsgemäßes Aroma

Ein grün-gelb eingefärbtes sprühgetrocknetes erfindungsgemäßes Aroma (enthaltend Maltodextrin (DE: 18-20), Dextrose, Gummi Arabicum, Aroma A1 aus Beispiel 2, (-)-Carvon, Farbstoff und das Antioxidans Ascorbylpalmitat) mit folgender Partikelgrößenverteilung wurde über eine Druckdüse hergestellt:
D (v 0,1): 26,8 Mikrometer,
D (v 0,5): 68,02 Mikrometer,
D (v 0,9): 126,4 Mikrometer
Beispiel F19: Instant-Getränkepulver mit sprühgetrocknetem erfindungsgemäßen Aroma

| | |
|---|---|
| Zucker (Saccharose) | 82,249 Gew.-% |
| Citronensäure | 11,58 Gew.-% |
| Trinatriumcitrat | 0,70 Gew.-% |
| Tricalciumphosphat | 0,60 Gew.-% |
| Vitamin C | 0,66 Gew.-% |
| Grindsted^{®} JU 543 Stabilizer System (Danisco) | 0,90 Gew.-% |
| Saccharin | 0,561 Gew.-% |
| Citronenaroma, sprühgetrocknet | 1,75 Gew.-% |
| Aroma, sprühgetrocknet, gemäß Beispiel F18 | 1,00 Gew.-% |

45 g dieses Instant-Getränkepulvers wurden in 1000 mL unter Rühren gelöst. Das erhaltene Getränk hatte einen erfrischenden, kühlenden Geschmack von Citrone und Pfefferminze.

### Beispiel F20: Hartkaramelle (hard boiled candy) mit erfindungsgemäßen Aroma

| | |
|---|---|
| Zucker (Saccharose) | 51,89 Gew.-% |
| Maissirup (Corn syrup), enthält Glucose und Fructose | 41,00 Gew.-% |
| Maltose | 3,00 Gew.-% |
| Palmkernöl | 0,90 Gew.-% |
| Citronensäure | 0,30 Gew.-% |
| Ginseng Extrakt | 0,40 Gew.-% |
| Blauer Farbstoff | 0,01 Gew.-% |
| Aroma A2 aus Beispiel 2 | 2,50 Gew.-% |

Zucker, Maissirup und Maltose wurden in Wasser gelöst, gekocht und vakuumiert. Anschließend wurden die restlichen Zutaten unter die gekochte Zuckermasse gezogen und bei Siedetemperatur homogenisiert. Nach Abkühlen wurden aus der resultierenden Masse Hartkaramellen geprägt. Die Hartkaramellen zeigten einen Restwassergehalt von etwa 2,5 Gew.-%.

### Beispiel F21: Halsbonbons mit flüssig-viskoser Kernfüllung (center-filled hard candy) mit erfindungsgemäßem Aroma

| | I (Gew.-%) | II (Gew.-%) |
|---|---|---|
| **Mischung A (Hülle)** (80% der Bonbons) | | |
| Zucker (Saccharose) | 58,12 | 49,37 |
| Glucosesirup (Feststoffgehalt 80%) | 41,51 | 49,37 |
| Aroma A1 aus Beispiel 2 | 0,17 | 0,25 |
| l-Menthol | 0,10 | - |
| Citronenöl | 0,10 | 0,10 |
| Citronensäure | - | 0,91 |
| Total: | 100 | 100 |

| **Mischung B (Kern)** (20% der Bonbons) | | |
|---|---|---|
| High Fructose Maissirup (Gehalt an festen Zuckern 85%, knapp 15% Wasser) | 84,38 | 84,36 |
| Glycerin | 15,0 | 15,0 |
| Lecithin | 0,02 | 0,02 |
| Zimtöl | - | 0,32 |
| Aroma A1 aus Beispiel 2 | 0,28 | - |
| Capsaicin | 0,05 | - |
| Vanillylalkohol-n-butylether | - | 0,10 |
| Roter Farbstoff, als 5%ige wässrige Lösung | 0,20 | 0,20 |
| Vanillin | 0,07 | - |
| Total | 100 | 100 |

In Anlehnung an die in US 6,432,441 (dort Beispiel 1) sowie die in US 5,458,894 bzw. US 5,002,791 beschriebenen Verfahren wurden Bonbons mit flüssigviskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Beispiel F22: Likör mit erfindungsgemäßem Aroma

Ein verzehrfertiges alkoholisches Getränk in Form eines nach Pfefferminz schmeckenden Likörs wurde hergestellt durch Mischen von 5 g erfindungsgemäßem Aroma A2 aus Beispiel 2 und 100 Litern einer grün eingefärbten alkoholisch-wässrigen Zuckerlösung, welche 18 Gew.-% Zucker (Saccharose) und 17,5 vol.-% Alkohol (Ethanol) enthielt.

### Beispiel F23: Erfrischungsgetränk mit erfindungsgemäßem Aroma

Ein verzehrfertiges nicht-alkoholisches Getränk in Form eines nach Pfefferminz schmeckenden Erfrischungsgetränks wurde hergestellt durch Mischen von 1 g erfindungsgemäßem Aroma A2 aus Beispiel 2 und 100 Litern einer gelbgrün eingefärbten 10,8 Gew.-%igen wässrigen Zuckerlösung.

## Patentansprüche

1. Verwendung von 4-Methyl-delta-hexalacton zum Vermitteln, Modifizieren und/oder Verstärken einer oder mehrerer Geruchs- oder Geschmackseindrücke aus der Gruppe bestehend aus Heu, Cumarin, Lacton und Mundfülle.

2. Parfümierter oder aromatisierter Artikel umfassend oder bestehend aus:
(a) 4-Methyl-delta-hexalacton,
(b) einer, mehreren oder sämtlichen Verbindungen ausgewählt aus der Gruppe bestehend aus
(i) Menthol,
(ii) (-)-Carvon
(iii) Menthon und
(iv) Isomenthon,
sowie vorzugsweise
(c) Nonenolid
und/oder
(d) Menthylacetat.

3. Artikel nach Anspruch 2, wobei der Artikel ausgewählt ist aus der Gruppe bestehend aus:
- Riech- oder Aromastoffkomposition, vorzugsweise Pfefferminz- oder Krauseminzaromastoffkomposition,
- der Ernährung dienende Zubereitung, vorzugsweise verzehrfertiges oder nicht verzehrfertiges Lebensmittel,
- der Mundpflege dienende Zubereitung und
- dem Genuss dienende Zubereitung.

4. Artikel nach Anspruch 2 oder 3, enthaltend eine Menge an 4-Methyl-delta-hexalacton, die ausreicht, um einen oder mehrere Geruchs- oder Geschmackseindrücke aus der Gruppe bestehend aus Heu, Cumarin, Lacton und Mundfülle zu vermitteln, zu modifizieren und/oder zu verstärken.

5. Artikel nach Anspruch 3 oder 4, **dadurch gekennzeichnet, dass** die Riech- oder Aromastoffkomposition eine Menge an 4-Methyl-delta-hexalacton im Bereich von 50 bis 8000 ppm, bevorzugt 100 bis 5000 ppm und besonders bevorzugt 250 bis 2000 ppm, bezogen auf die Gesamtmenge der Riech- oder Aromastoffkomposition, umfasst.

6. Artikel nach einem der Ansprüche 3 bis 5, **dadurch gekennzeichnet, dass** die Riech- oder Aromastoffkomposition oder der Ernährung, der Mundpflege oder dem Genuss dienende Zubereitung eine Menge an einer oder mehreren Verbindungen ausgewählt aus der Gruppe bestehend aus
(i) Menthol,
(ii) (-)-Carvon,
(iii) Menthon und
(iv) Isomenthon, umfasst,
die ausreicht, um in der Riech- oder Aromastoffkomposition oder der Zubereitung eine dominierende Pefferminz- und/oder Krauseminz-Geruchs- oder Geschmacksnote zu erzeugen.

7. Artikel nach einem der Ansprüche 3 bis 6, **dadurch gekennzeichnet, dass** die Riech- oder Aromastoffkomposition
(a) 4-Methyl-delta-hexalacton in einer Menge von 50 bis 8000 ppm, bevorzugt
100 bis 5000 ppm und besonders bevorzugt 250 bis 2000 ppm, sowie
(b) (i) Menthol, vorzugsweise I-Menthol, in einer Menge von 1 bis 80 Gew.-%, bevorzugt 5 bis 70 Gew.-%,
und/oder
(ii) (-)-Carvon in einer Menge von 1 bis 80 Gew.-%, bevorzugt 5 bis 80 Gew.-%,
und/oder
(iii) Menthon, vorzugsweise (-)-Menthon,
und/oder
(iv) Isomenthon, vorzugsweise (+)-Isomenthon, in einer Gesamtmenge von 0.5 bis 60 Gew.-%, bevorzugt 1 bis 50 Gew.-%,
sowie vorzugsweise
(c) Nonenolid in einer Menge von 50 bis 8000 ppm, bevorzugt 100 bis 5000 ppm und besonders bevorzugt 250 bis 2000 ppm
und/oder
(d) Menthylacetat, vorzugsweise (-)-Menthylacetat in einer Menge von 1 bis 20 Gew.-%, bevorzugt 2 bis 12 Gew.-%, besonders bevorzugt 3 bis 10 Gew.-%, umfasst,
wobei die Mengenangaben jeweils bezogen sind auf die Gesamtmenge der Riech- oder Aromastoffkomposition.

8. Artikel nach einem der Ansprüche 3 bis 6, **dadurch gekennzeichnet, dass** die der Ernährung, der Mundpflege oder dem Genuss dienende Zubereitung
- 4-Methyl-delta-hexalacton in einer Menge im Bereich von 0,05 bis 80 ppm, bevorzugt im Bereich von 0,1 bis 50 ppm und besonders bevorzugt im Bereich von 0,25 bis 20 ppm
oder
- eine Riech- oder Aromastoftkomposition nach einem der Ansprüche 3 bis 7 in einer Menge von 0,05 bis 50 Gew.-%, bevorzugt im Bereich von 0,05 bis 6 Gew.-%, vorzugsweise im Bereich von 0,1 bis 3 Gew.-%, umfasst,
bezogen auf das Gesamtgewicht der Zubereitung.

9. Artikel nach Anspruch 8, wobei die Zubereitung gebrauchsfertig ist und vorzugsweise
(A) eine verzehrfertige und dabei zuckerfreie, zuckerreduzierte oder zuckerhaltige Süßware, insbesondere in Form von: Schokolade, gefüllten Schokolade (beispielsweise mit aromatisierter Fondant-Masse, z.B. des Typs After-Eight), Schokoladenriegelprodukt, Fruchtgummi, Hart- oder Weichkaramelle, Kaubonbon, Zuckerperle, Lutscher, Kapsel (vorzugsweise nahtlose Kapsel, bevorzugt zum Direktverzehr, vorzugsweise mit einer Umhüllung basierend auf Gelatine und/oder Alginat), Kaugummi (z.B. in Form von Streifen, Komprimaten, Pellets, Kissen, Kugeln, Hohlkugeln)
oder
(B) ein Mundpflegeprodukt (Mundhygieneprodukt), insbesondere in Form von: Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide, oder Zahnpflegekaugummi
oder
(C) ein alkoholisches oder nicht-alkoholisches Getränk
ist.

10. Verfahren zum Vermitteln, Modifizieren und/oder Verstärken einer oder mehrerer Geruchs- oder Geschmackseindrücke aus der Gruppe Heu, Cumarin, Lacton und Mundfülle, wobei 4-Methyl-delta-hexalacton oder eine 4-Methyl-delta-hexalacton umfassende Riech- oder Aromastoffkomposition, vorzugsweise nach einem der Ansprüche 3 bis 7, mit einem Erzeugnis in Kontakt gebracht oder gemischt wird.

11. Verfahren zur Herstellung einer Riech- oder Aromastoffkomposition, mit folgendem Schritt:
- Vermischen von
4-Methyl-delta-hexafacton mit üblichen Bestandteilen einer Riech- oder Aromastoffkomposition,
wobei das 4-Methyl-delta-hexalacton in einer Menge eingesetzt wird, die ausreicht, um in der Riech- oder Aromastoffkomposition einen oder mehrere Geruchs- oder Geschmackseindrücke aus der Gruppe bestehend aus Heu, Cumarin, Lacton und Mundfülle zu vermitteln, zu modifizieren und/oder zu verstärken.

12. Verfahren nach Anspruch 11, wobei die Riech- oder Aromastoffkomposition eine Riech- oder Aromastoffkomposition nach einem der Ansprüche 3 bis 7 ist und
(a) 4-Methyl-delta-hexalacton
mit
(b) einer, mehreren oder sämtlichen Verbindungen ausgewählt aus der Gruppe bestehend aus
(i) Menthol,
(ii) (-)-Carvon
(iii) Menthon und
(iv) Isomenthon,
sowie vorzugsweise
(c) Nonenolid
und/oder
(d) Menthylacetat
vermischt wird.

## Claims

1. Use of 4-methyl-delta-hexalactone for imparting, modifying and/or enhancing one or more odour or taste impressions from the group consisting of hay, coumarin, lactone and mouth fullness.

2. A perfumed or aromatised article comprising or consisting of:
(a) 4-methyl-delta-hexalactone,
(b) one, a plurality of or all the compounds selected from the group consisting of
(i) menthol,
(ii) (-)-carvone;
(iii) menthone and
(iv) isomenthone,
preferably together with
(c) nonenolide and/or
(d) menthyl acetate.

3. An article according to claim 2, wherein the article is selected from the group consisting of;
- an odoriferous or aroma substance composition, preferably a peppermint or spearmint aroma substance composition,
- a preparation serving for nutrition, preferably a foodstuff which is or is not ready-to-consume,
- a preparation serving for oral care and
- a preparation serving for pleasure.

4. An article according to claim 2 or claim 3, containing a quantity of 4-methyl-delta-hexalactone which is sufficient to impart, modify and/or enhance one or more odour- or taste impressions from the group consisting of hay, coumarin, lactone and mouth fullness.

5. An article according to claim 3 or claim 4, **characterised in that** the odoriferous or aroma substance composition comprises a quantity of 4-methyl-delta-hexalactone in the range from 50 to 8000 ppm, preferably 100 to 5000 ppm and particularly preferably 250 to 2000 ppm, relative to the total quantity of the odoriferous or aroma substance composition.

6. An article according to any one of claims 3 to 5, **characterised in that** the odoriferous or aroma substance composition or preparation serving for nutrition, oral care or pleasure comprises a quantity of one or more compounds selected from the group consisting of
(i) menthol,
(ii) (-)-carvone,
(iii) menthone and
(iv) isomenthone,
which is sufficient to produce a dominant peppermint and/or spearmint odour or flavour note in the odoriferous or aroma substance composition or the preparation.

7. An article according to any one of claims 3 to 6, **characterised in that** the odoriferous or aroma substance composition comprises
(a) 4-methyl-delta-hexalactone in a quantity of 50 to 8000 ppm, preferably of 100 to 5000 ppm and particularly preferably of 250 to 2000 ppm, together with
(b)
and/or
(i) menthol, preferably 1-menthol, in a quantity of 1 to 80 wt.%, preferably of 5 to 70 wt.%,
(ii) (-)-carvone in a quantity of 1 to 80 wt.%, preferably of 5 to 80 wt.%,
and/or
(iii) menthone, preferably (-)-menthone, and/or
(iv) isomenthone, preferably (+)-isomenthone, in a total quantity of 0.5 to .60 wt.%, preferably of 1 to 50 wt.%, preferably together with
(c) nonenolide in a quantity of 50 to 8000 ppm, preferably of 100 to 5000 ppm and particularly preferably of 250 to 2000 ppm
and/or
(d) menthyl acetate, preferably (-)-menthyl acetate, in a quantity of 1 to 20 wt.%, preferably of 2 to 12 wt.%, particularly preferably of 3 to 10 wt.%,
wherein the stated quantities are in each case relative to the total quantity of the odoriferous or aroma substance composition.

8. An article according to any one of claims 3 to 6, **characterised in that** the preparation serving for nutrition, oral care or pleasure comprises
- 4-methyl-delta-hexalactone in a quantity in the range from 0.05 to 80 ppm, preferably in the range from 0.1 to 50 ppm and particularly preferably in the range from 0.25 to 20 ppm
or
- an odoriferous or aroma substance composition according to any one of claims 3 to 7 in a quantity of 0.05 to 50 wt.%, preferably in the range from 0.05 to 6 wt.%, preferably in the range from 0.1 to 3 wt.%,
relative to the total weight of the preparation.

9. An article according to claim 8, wherein the preparation is ready-to-use and is preferably
(A) a ready-to-consume and sugar-free, reduced-sugar or sugar-containing item of confectionery, in particular in the form of: chocolate, filled chocolate (for example comprising an aromatised fondant mass, for example of the After-Eight type), chocolate bar product, fruit gums, hard or soft caramels, chewable sweets, sugar pearls, lollipops, capsules (preferably seamless capsules, preferably for direct consumption, preferably with a shell based on gelatin and/or alginate), chewing gum (for example in the form of strips, compressed tablets, pellets, cushions, spheres, hollow spheres)
or
(B) an oral care product (oral hygiene product), in particular in the form of: toothpaste, tooth cream, tooth gel, tooth powder, dental cleaning liquid, dental cleaning foam, mouthwash, tooth cream and mouthwash as a 2-in-1 product, boiled sweet, mouthspray, dental floss or dental care chewing gum
or
(C) an alcoholic or non-alcoholic beverage.

10. A method for imparting, modifying and/or enhancing one or more odour or taste impressions from the group hay, coumarin, lactone and mouth fullness, wherein 4-methyl-delta-hexalactone or an odoriferous or aroma substance composition comprising 4-methyl-delta-hexalactone, preferably according to any one of claims 3 to 7, is brought into contact or mixed with a product.

11. A method for producing an odoriferous or aroma substance composition comprising the following step:
- mixing
4-methyl-delta-hexalactone with conventional constituents of an odoriferous or aroma substance composition,
wherein the 4-methyl-delta-hexalactone is used in a quantity which is sufficient to impart, modify and/or enhance one or more odour or taste impressions from the group consisting of hay, coumarin, lactone and mouth fullness in the odoriferous or aroma substance composition.

12. A method according to claim 11, wherein the odoriferous or aroma substance composition is an odoriferous or aroma substance composition according to any one of claims 3 to 7 and
(a) 4-methyl-delta-hexalactone is mixed with
(b) one, a plurality of or all the compounds selected from the group consisting of
(i) menthol,
(ii) (-)-carvone
(iii) menthone and
(iv) isomenthone,
preferably together with
(c) nonenolide
and/or
(d) menthyl acetate.

## Revendications

1. Utilisation de la 4-méthyl-delta-hexalactone pour conférer, modifier et/ou renforcer une ou plusieurs impressions olfactives ou gustatives du groupe consistant en foin, coumarine, lactone et plénitude en bouche.

2. Article parfumé ou aromatisé comprenant ou consistant en :
(a) 4-méthyl-delta-hexalactone,
(b) un, plusieurs ou tous les composés choisis dans le groupe consistant en
(i) menthol,
(ii) (-)-carvone,
(iii) menthone et
(iv) isomenthone,
ainsi que, de préférence
(c) nonénolide
et/ou
(d) acétate de menthyle.

3. Article selon la revendication 2 où l'article est choisi dans le groupe consistant en
- une composition de substances odorantes ou aromatiques, de préférence une composition de substances odorantes de menthe poivrée ou de menthe crépue,
- une préparation servant à l'alimentation, de préférence un aliment prêt à consommer ou non prêt à consommer,
- une préparation servant aux soins buccaux et
- une préparation servant au plaisir.

4. Article selon la revendication 2 ou 3 contenant une quantité de 4-méthyl-delta-hexalactone qui suffit à conférer, à modifier et/ou à renforcer une ou plusieurs impressions olfactives ou gustatives du groupe consistant en foin, coumarine, lactone et plénitude en bouche.

5. Article selon la revendication 3 ou 4 **caractérisé en ce que** la composition de substances odorantes ou aromatiques comprend une quantité de 4-méthyl-delta-hexalactone dans la plage de 50 à 8 000 ppm, de préférence de 100 à 5 000 ppm et de manière particulièrement préférée de 250 à 2 000 ppm, par rapport à la quantité totale de composition de substances odorantes ou aromatiques.

6. Article selon l'une des revendications 3 à 5 **caractérisé en ce que** la composition de substances odorantes ou aromatiques ou la préparation servant à l'alimentation, aux soins buccaux ou au plaisir comprend une quantité d'un ou plusieurs composés choisis dans le groupe consistant en
(i) menthol,
(ii) (-)-carvone,
(iii) menthone et
(iv) isomenthone
qui est suffisante pour produire dans la composition de substances odorantes ou aromatiques ou dans la préparation une note olfactive ou gustative de menthe poivrée et/ou de menthe crépue dominante.

7. Article selon l'une des revendications 3 à 6 **caractérisé en ce que** la composition de substances odorantes ou aromatiques comprend
(a) de la 4-méthyl-delta-hexalactone en une quantité de 50 à 8 000 ppm, de préférence de 100 à 5 000 ppm et de manière particulièrement préférée de 250 à 2 000 ppm,
ainsi que
(b)
(i) du menthol, de préférence du I-menthol, en une quantité de 1 à 80 % en masse, de préférence de 5 à 70 % en masse,
et/ou
(ii) de la (-)-carvone en une quantité de 1 à 80 % en masse, de préférence de 5 à 80 % en masse,
et/ou
(iii) de la menthone, de préférence de la (-)-menthone, et/ou
(iv) de l'isomenthone, de préférence de la (+)-isomenthone, en une quantité totale de 0,5 à 60 % en masse, de préférence de 1 à 50 % en masse,
ainsi que de préférence
(c) du nonénolide en une quantité de 50 à 8 000 ppm, de préférence de 100 à 5 000 ppm et de manière particulièrement préférée de 250 à 2 000 ppm
et/ou
(d) de l'acétate de menthyle, de préférence de l'acétate de (-)-menthyle en une quantité de 1 à 20 % en masse, de préférence de 2 à 12 % en masse, de manière particulièrement préférée de 3 à 10 % en masse,
où les indications de quantités sont rapportées dans chaque cas à la quantité totale de la composition de substances odorantes ou aromatiques.

8. Article selon l'une des revendications 3 à 6 **caractérisé en ce que** la préparation servant à l'alimentation, aux soins buccaux ou au plaisir comprend
- de la 4-méthyl-delta-hexalactone en une quantité dans la plage de 0,05 à 80 ppm, de préférence dans la plage de 0,1 à 50 ppm et de manière particulièrement préférée dans la plage de 0,25 à 20 ppm,
ou
- une composition de substances odorantes ou aromatiques selon l'une des revendications 3 à 7 en une quantité de 0,05 à 50 % en masse, de préférence dans la plage de 0,05 à 6 % en masse, de préférence dans la plage de 0,1 à 3 % en masse,
par rapport à la masse totale de la préparation.

9. Article selon la revendication 8 où la préparation est prête à l'emploi et est de préférence
(A) une confiserie prête à consommer et sans sucre, réduite en sucre ou contenant du sucre, en particulier sous forme de : chocolat, chocolat fourré (par exemple avec une masse fondante aromatisée, par exemple du type afker-eight), un produit de type barre chocolatée, de la gomme de fruits, du caramel dur ou mou, un bonbon à mâcher, une perle de sucre, une sucette, une capsule (de préférence une capsule sans joint, de préférence pour la consommation directe, de préférence avec un enrobage à base de gélatine et/ou d'alginate), de la gomme à mâcher (par exemple sous forme de bandes, de comprimés, de pastilles, de coussins, de billes, de billes creuses)
ou
(B) un produit pour les soins buccaux (produit pour l'hygiène buccale), en particulier sous forme de : pâte dentifrice, crème dentifrice, gel dentifrice, poudre dentifrice, liquide dentifrice, mousse dentifrice, eau dentifrice, crème dentifrice et eau dentifrice sous forme d'un produit 2 en 1, bonbons à sucer, spray buccal, soie dentaire ou gomme à mâcher pour les soins dentaires
ou
(C) une boisson alcoolique ou non alcoolique.

10. Procédé pour conférer, modifier et/ou renforcer une ou plusieurs impressions olfactives ou gustatives du groupe foin, coumarine, lactone et plénitude en bouche, où de la 4-méthyl-delta-hexalactone ou une composition de substances odorantes ou aromatiques comprenant de la 4-méthyl-delta-hexalactone, de préférence selon l'une des revendications 3 à 7, est mise en contact ou mélangée avec un produit.

11. Procédé pour produire une composition de substances odorantes ou aromatiques comportant l'étape suivante :
- mélange de
4-méthyl-delta-hexalactone avec des constituants courants d'une composition de substances odorantes ou aromatiques,
où la 4-méthyl-delta-hexalactone est utilisée en une quantité qui est suffisante pour conférer, pour modifier et/ou pour renforcer dans la composition de substances odorantes ou aromatiques une ou plusieurs impressions olfactives ou gustatives du groupe consistant en foin, coumarine, lactone et plénitude en bouche.

12. Procédé selon la revendication 11 où la composition de substances odorantes ou aromatiques est une composition de substances odorantes ou aromatiques selon l'une des revendications 3 à 7 et
(a) la 4-méthyl-delta-hexalactone est mélangée avec
(b) un, ou plusieurs ou tous les composés choisis dans le groupe consistant en
(i) menthol,
(ii) (-)-carvone,
(iii) menthone et
(iv) isomenthone, ainsi que de préférence
(c) nonénolide
et/ou
(d) acétate de menthyle.
